# EUROPEAN PATENT APPLICATION

(11) **EP 4 686 756 A1**
(43) Date of publication of application: **04.02.2026**
(21) Application number: 24191874.7
(22) Date of filing: 30.07.2024
(51) Int. Cl.: C12N 15/113

(54) **METHOD FOR TARGETING AND MAPPING OF ESSENTIAL PHAGE GENES AND USES THEREFOR**

(71) Applicant: Helmholtz-Zentrum für Infektionsforschung GmbH, 38124 Braunschweig (DE); Julius-Maximilians-Universität Würzburg, in Vertretung des Freistaates Bayern, 97070 Würzburg (DE)
(72) Inventor: Vogel, Jörg, 97074 Würzburg (DE); Gerovac, Milan, 97080 Würzburg (DE); Durica-Mitic, Svetlana, 1140 Wien (AT)
(74) Representative: Virnekäs, Bernhard

(57) **Abstract**

This invention relates to a novel method for the targeting and mapping of phage genes that are essential or important for phage replication in their bacterial host cells, to novel methods for preventing or inhibiting the formation of progeny particles of a phage from a bacterium infected with said phage, and to novel methods for promoting the formation of phage progeny particles from a bacterium.

## Description

### FIELD OF THE INVENTION

This invention relates to a novel method for the targeting and mapping of phage genes that are essential or important for phage replication in their bacterial host cells, to novel methods for preventing or inhibiting the formation of progeny particles of a phage from a bacterium infected with said phage, and to novel methods for promoting the formation of phage progeny particles from a bacterium.

### BACKGROUND OF THE INVENTION

Bacteriophages have become important study systems in molecular biology, biotechnology and medicine. This growing interest is driven by the richness of unexplored genes that emerge in the phage-host conflict (Bernheim & Sorek 2020, Mayo-Muñoz et al. 2024). Targeted mapping and characterization of genes that act at the phage-host interface is key to understanding the major molecular players in a phage's infection cycle and its ability to counter host defense, but the diversity and genetic intractability of phages and their hosts creates a major challenge. While early work involved temperature-sensitive or other conditional mutants (Ofir & Sorek 2018), chemical mutagenesis (Robins et al. 2013) and small RNAs (Sturino & Klaenhammer 2002), recent studies increasingly used CRISPR-Cas technology to inhibit phage gene expression (McDonnell et al. 2018, Piya et al. 2023, Adler et al. 2023, Sprenger et al. 2024). Notwithstanding the success of these techniques, a main barrier remains: most of these approaches require genetic manipulation of the bacterial hosts. Yet, many phage hosts cannot be transformed or conjugated (Marsh et al. 2023) due to defence systems that target foreign DNA (Bernheim & Sorek 2022, Mayo-Muñoz et al. 2023). In addition, phages encode anti-CRISPR proteins or non-coding RNAs that can neutralise Cas enzymes (Bondy-Denomy et al. 2013, Camara-Wilpert et al. 2023). As a result, it remains difficult to map and characterise genes in the vast majority of phages, among them as a particularly interesting example the intensely studied jumbo phages. Up to now this knockdown approach was only used to target the Chimallivirus Goslar, which infects E. *coli* (Adler et al. 2023). Application to Pseudomonas-infecting phages has not been possible due to the challenge of transferring the Cas system to this bacterial species. The major drawback of these approaches is that they require genetic manipulation of the bacterial hosts. Yet, many phage hosts cannot be transformed or conjugated (Marsh, Kirk, Ley 2023). Moreover, bacteria frequently encode anti-CRISPR proteins that neutralize Cas enzymes (Katz et al. 2023).

An alternative approach to silence transcripts is provided by short antisense oligomers (ASOs) that interfere with translation through the binding of the ribosome binding site (RBS) or start codon are (reviewed in Kole & Krainer & Altman 2012, Pifer & Greenberg 2020). ASOs have previously been used in cell-free systems to manipulate phage production (Vogele et al. 2021), but they have not been applied before to inhibit phage replication in their bacterial hosts.

In that context, a number of phage biology-specific features are of particular concern. First, in contrast to the translation of the bacterial mRNA reservoir, the production of individual phage-derived mRNA during phage infection and replication is substantially higher than the one from the host, thus rendering it less likely that sufficient amounts of inhibitor or modifier molecules, such as ASOs, could be made available for inhibition or modification of phage-derived mRNA translation. Second, the tight regulation and rapid and concerted handover between transcription and translation of the newly formed phage-derived mRNA in phage-infected cells renders it less likely that an inhibitor can efficiently attach to its target on the mRNA and thus block, or at least modulate, translation, and ultimately phage replication. Third, phages have developed a number of mechanisms that block bacterial control mechanisms and render phage-infected cells unpredictable in their responses, for example, at the level of mRNA surveillance that aim at degradation of e.g. not translated mRNAs, so that even if a temporary stay of translation could be envisaged for phage-derived mRNAs by using an ASO-based inhibition approach, a medium- to long-term success appears to be rather unlikely because the mRNAs may accumulate over time and exceed the inhibitory capacity of ASOs.

Thus, there is an unmet need for the development of methods for identifying genes and/or proteins that are essential or important for the replication of phages in bacteria, and methods for addressing and influencing the replication of phages in bacteria by inhibiting phage genes.

### OBJECTS OF THE INVENTION

It was thus an object of the invention to provide a novel method for the targeting and mapping of phage and/or bacterial genes that are essential or important for phage replication in their bacterial host cells, to novel methods for preventing or inhibiting the formation of progeny particles of a phage from a bacterium infected with said phage, and to novel methods for promoting the formation of phage progeny particles from a bacterium.

### SUMMARY OF THE INVENTION

Surprisingly, it was found that by using a combination of a cell-penetrating peptide and a short antisense oligonucleotide, and by identifying and optimizing certain parameters for performing the use of such combinations, genes and proteins that are essential or important for phage replication in a bacterial host cell can be targeted so that phage replication can be modulated.

Thus, in a first aspect, the present invention relates to a method for assaying for the relevance of a protein of a phage for the replication of said phage in a bacterium, comprising the steps of (a) contacting said bacterium either before, concomitant with or after infection with said phage, in particular before infection with said phage, with at least one compound CPP-ASO^{p}, wherein CPP is a cell-penetrating peptide and ASO^{p} is an antisense construct that is specifically binding to an mRNA coding for said protein, and (b) evaluating at least one parameter that is a measure for said replication.

In a second aspect, the present invention relates to a method for assaying for the relevance of a protein of a bacterium for the replication of a phage in said bacterium, comprising the steps of (a) contacting said bacterium either before, concomitant with or after infection with said phage with at least one compound CPP-ASOb, wherein CPP is a cell-penetrating peptide and ASOb is an antisense construct that is specifically binding to an mRNA coding for said protein, and (b) evaluation at least one parameter that is a measure for said replication.

In a third aspect, the present invention relates to a method for inhibiting the formation of progeny particles of a phage from a bacterium infected with said phage, comprising the step of contacting said bacterium with at least one compound CPP-ASO^{p}, wherein CPP is a cell-penetrating peptide and ASO^{p} is an antisense construct that is specifically binding to an mRNA coding for a protein of said phage that is essential or important for phage replication, and/or at least one compound CPP-ASO^{b}, wherein ASO^{b} is an antisense construct that is specifically binding to an mRNA coding for a protein of said bacterium that is essential or important for phage replication.

In a fourth aspect, the present invention relates to a method for preventing the formation of phage progeny particles from a bacterium at risk of being infected with said phage, comprising the step of contacting said bacterium with at least one compound CPP-ASO^{p}, wherein CPP is a cell-penetrating peptide and ASO^{p} is an antisense construct that is specifically binding to an mRNA coding for a protein of said phage that is essential or important for phage replication, and/or at least one compound CPP- ASO^{b}, wherein ASO^{b} is an antisense construct that is specifically binding to an mRNA coding for a protein of said bacterium that is essential or important for phage replication.

In a fifth aspect, the present invention relates to a method for promoting the formation of phage progeny particles from a bacterium comprising the steps of (a) contacting said bacterium with at least one compound CPP- ASO^{b}, wherein CPP is a cell-penetrating peptide and ASO^{b} is an antisense construct that is specifically binding to an mRNA coding for a protein essential or important for said bacterium's defence system directed against replication of said phage in said bacterium; and (b) infecting said bacterium with said phage.

In a sixth aspect, the present invention relates to a method for promoting the formation of phage progeny particles from a bacterium infected with, or otherwise comprising, a phage, in particular a prophage, comprising the steps of (a) contacting said bacterium with at least one compound CPP-ASO^{p}, wherein CPP is a cell-penetrating peptide and ASO^{p} is an antisense construct that is specifically binding to an mRNA coding for a protein repressing, preventing or delaying replication of said phage in said bacterium.

In a seventh aspect, the present invention relates to a compound CPP-ASO^{p}, wherein CPP is a cell-penetrating peptide and ASOp is an antisense construct that is specifically binding to an mRNA coding for a protein repressing, preventing or delaying replication of a phage, in particular a lytic phage, in a bacterium for use in the treatment of a patient suffering from an infection with said bacterium.

### FIGURES

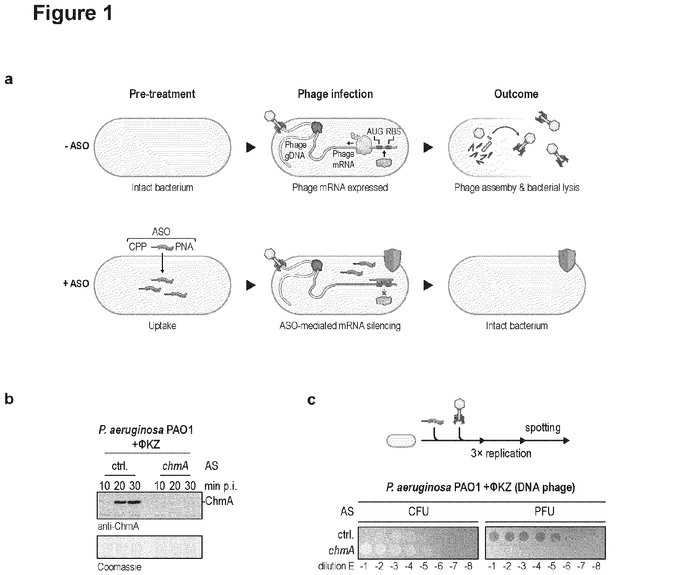

**Figure 1** shows that ASOs prevent phage replication in bacteria and genetically intractable strains. **a.** Antisense oligomers (ASO) are taken up by the bacterial cell mediated by cell penetrating peptides (CPPs). After phage infection, ASOs bind specific phage transcripts at the ribosome binding site (RBS) or the start codon (AUG) and prevent translation. If the protein encoded by the target phage mRNA is essential or important for phage replication, no phage progeny is formed, **b.** PAO1 cells were pretreated with an ASO against *chmA* or a non-targeting control ASO (ctrl.). After ΦKZ infection with a MOI of 5, cells were harvested at the indicated time points and ChmA levels were determined by immunoblotting. AS: ASO to the indicated transcript. **c.** PAO1 cells were pretreated with an ASO against *chmA* or a non-targeting control. After ΦKZ infection at MOI 0.0001, cells were incubated to allow three rounds of replication, which leads to nearly complete lysis of cells. The resulting phage-cell suspension was spotted on LB plates to assess colony forming units (CFUs) and on LB plates with a lawn of PAO1 cells to assess plaque forming units (PFUs). A high number of phages leads to lysis of nearby bacterial cells after spotting, leading to complete lack of CFUs at low dilutions.

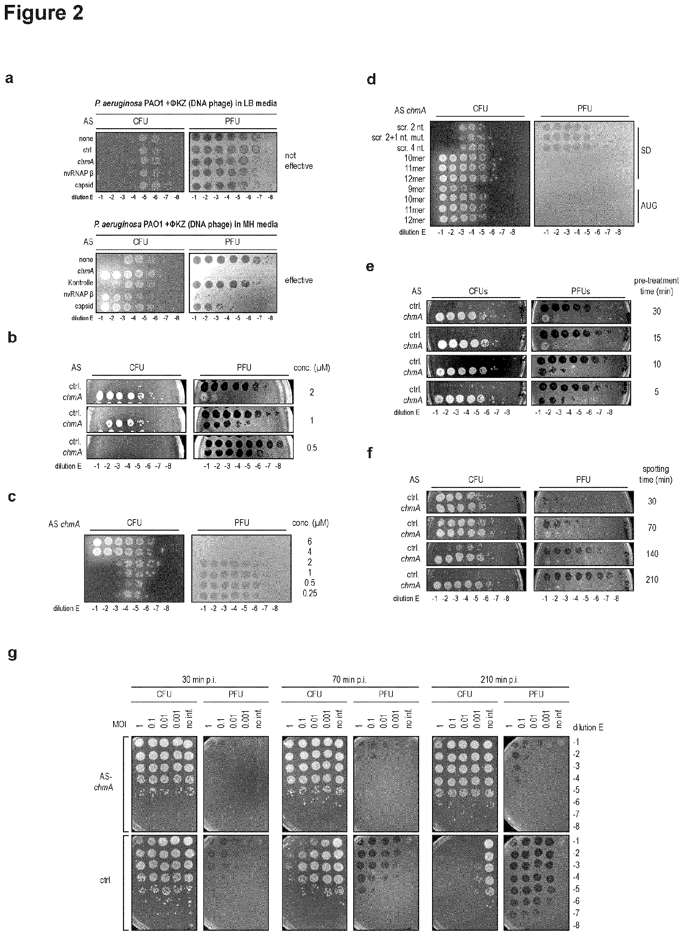

**Figure 2** shows the Optimization of ASO treatment. **a.** Effective media conditions for ASO treatment were determined by pretreatment of cells for 30 min with different ASOs in LB and MH media. Subsequently, cells were infected with ΦKZ, and incubated for 30 min, which was followed by CFU/PFU readout. **b.** Effective ASO concentration was determined by pretreating cells for 30 min with different ASO concentrations as indicated. Subsequently, cells were infected with ΦKZ, and incubated for 30 min, which was followed by CFU/PFU readout. **c.** Effective ASO concentration was determined by pretreating cells for 30 min with different ASO concentrations as indicated. Subsequently, cells were infected with ΦKZ, allowed to replicate for 3 h, which was followed by CFU/PFU readout. **d.** ASOs of different lengths and scrambled variants were tested. For the scrambled variants, two (2x) or four (4x) nucleotides were changed in their position, 3x represents 2x with one additional mutation; all changes were made in the central part of the ASO-sequence. ASOs were added at 6 µM. Subsequently, cells were infected with ΦKZ, allowed to replicate for 3 h, which was followed by CFU/PFU readout. SD Shine-Dalgarno sequence; AUG start codon. **e.** ASO pre-treatment time was tested at 5, 10, 15, and 30 min. **f.** Sampling time after infection was tested at below one replication round (30 min) and at one (70 min), two (140 min), and three (210 min) replication rounds, followed by CFU/PFU readout. **g.** MOI used for infection was tested at 1, 0.1, 0.01, and 0.001.

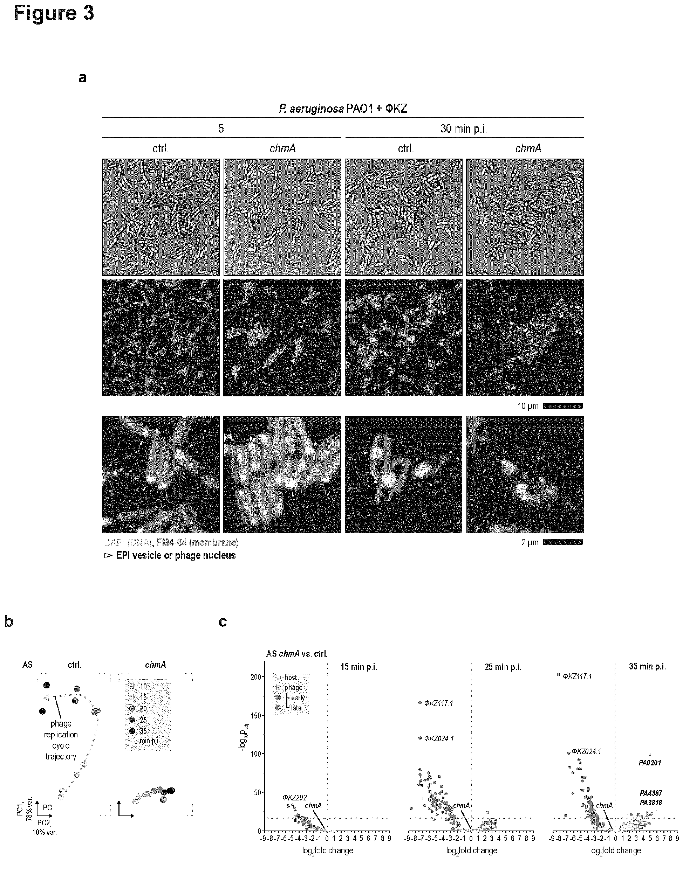

**Figure 3** shows that phage replication states can be arrested to study cellular structures and host response at the transcriptome level. **a.** PAO1 was treated with ASOs against *chmA* or a non-targeting control followed by imaging. Membranes are stained with FM4-64, DNA is stained with DAPI. **b.** ChmA was inhibited in ΦKZ-infected PAO1 cells and the transcriptome was sequenced at the indicated time points. To reduce the high-dimensional dataset, we projected the dataset based on transcript abundance of each annotated gene on two dimensions via principal component (PC) analysis. Each dot represents a replicate, time points are shaded in grey ctrl., non-targeting control ASO. **c.** Transcript enrichment between ASO-knockdown vs non-targeting control ASO (ctrl.) is depicted in a volcano plot at indicated time points. Duplicates were merged by geometrical averaging and P-values were calculated by the Wald test using DESeq2.

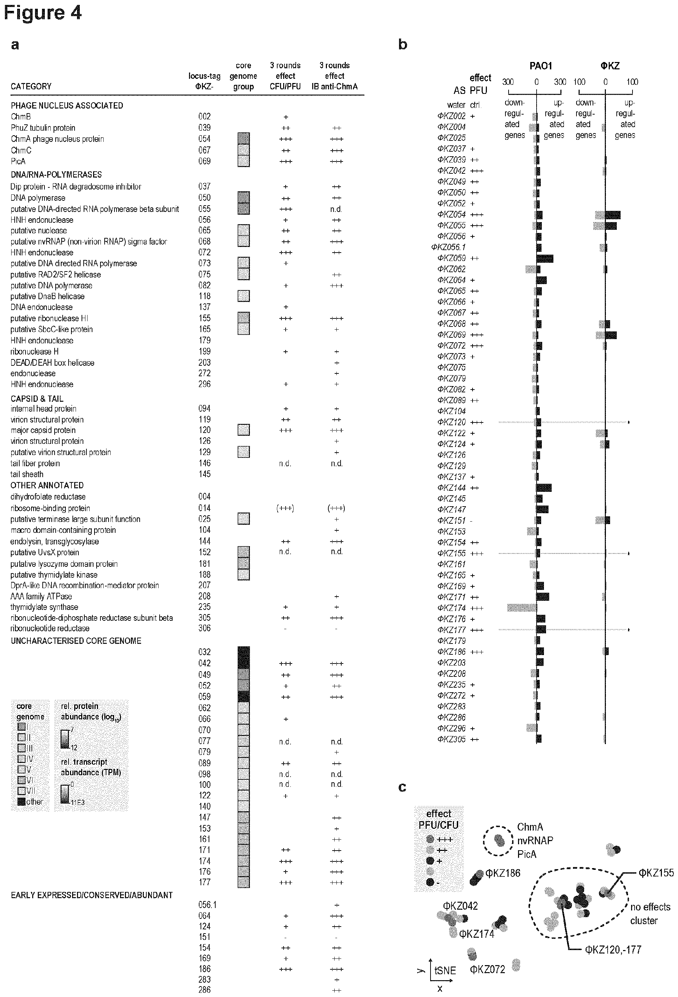

**Figure 4** shows that ASO-based screen identified a replication factor that is essential or important for ΦKZ replication. **a.** PAO1 cells were treated with a non-targeting control ASO (ctrl.) or ASOs against ΦKZ155 or ChmA and infected with ΦKZ. Cells were chemically crosslinked after 35 min p.i. and stained with DAPI and FM4-64 to visualise DNA and membranes followed by imaging. **b.** RNA was 5'-[³²P]-phosphorylated and optionally duplexed with complementary DNA (bottom). ΦKZ155 and the catalytically dead D102N mutant were produced by *in vitro* translation and added to the oligomer for the cleavage reaction. Subsequently, the oligomers were analysed on an Urea-PAGE gel and autoradiography. **c.** PAO1 cells were treated as in (a) and the cell culture was harvested at indicated time points for DNA extraction. The samples were dot blotted, RNA was eliminated by alkaline treatment. The phage genomic DNA was detected with a radiolabeled oligo probe JVO-23213 (complementary to the *chmA* open reading frame) and autoradiography.

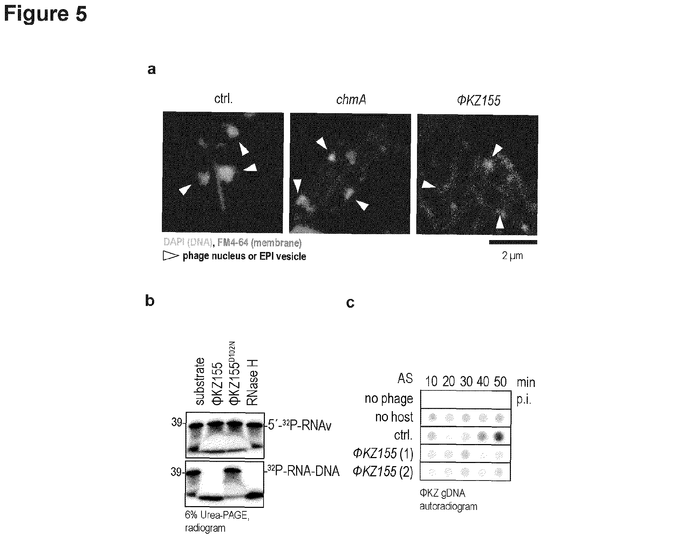

**Figure 5** shows that several core genes are important for ΦKZ replication. a. The impact of phage gene knockdown upon ASO treatment was evaluated by CFU/PFU determination and immunoblot detection of ChmA protein after three rounds of replication. The associations of targets to core genome blocks in *Chimalliviridae* (Prichard et al. 2023), transcript abundance at 10 min p.i., and average protein abundance at 10, 20, 30 min p.i. based on a previously published data set (Gerovac et al. 2024) is also shown. The CFU/PFU effect of each respective knockdown is depicted as no effect (), weak (+), effective (++), very effective plaque reduction (+++) and increased plaque levels (-). Minimum two ASOs were tested, toxic ones omitted, the stronger effect is represented. n.d. not determined due to e.g. both ASOs were toxic. **b.** PAO1 cells were treated with ASOs against the indicated phage transcripts, infected with ΦKZ at a MOI of 5 and the transcriptome was analysed by RNA-sequencing at 30 min p.i.. Bars indicate the counts of ΦKZ (top) or PAO1 genes (bottom) that were affected in abundance (Log2FC>2, <-2, >100 reads in sum over all knockdown experiments at 30 min p.i.). For each target gene one replicate was sequenced. * indicates three genes whose knockdown led to a strong reduction in plaque formation but had no effect on the phage transcriptome. **c.** Log2FC values for each ΦKZ gene from **(b)** were clustered by tSNE, and clusters were indicated by a dashed line.

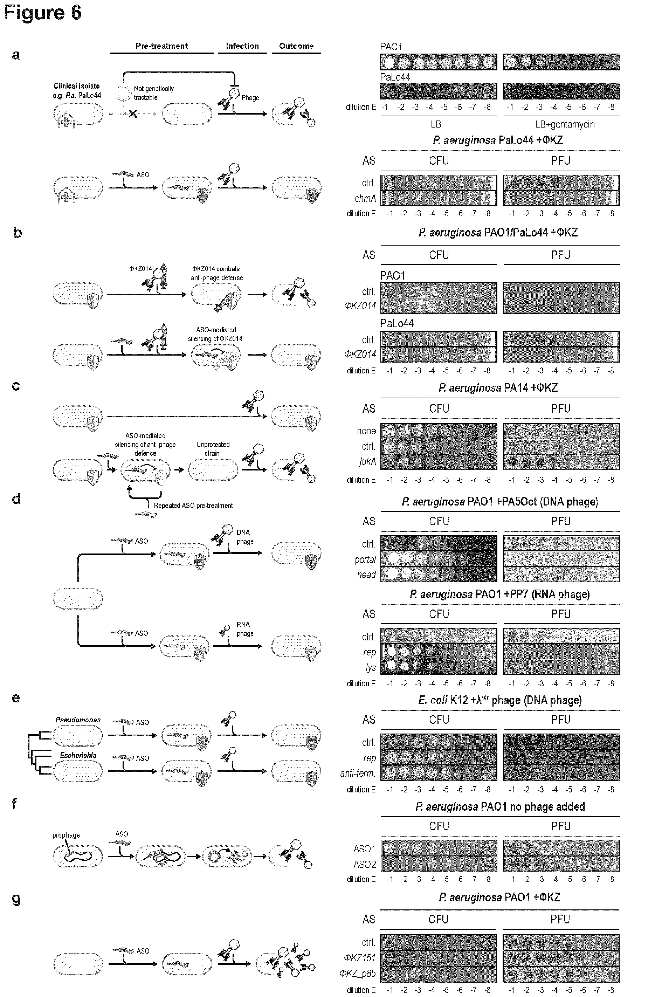

**Figure 6** shows the versatility of ASOs to phages. (Left) Schematic representation of potential ASO applications, such as targeting clinical isolates that are often genetically intractable **(a),** protecting bacteria from phage infection by silencing phage genes that overcome an anti-phage defense system **(b),** sensitising bacteria to phage infection by silencing a bacterial anti-phage defense system (c), targeting other jumbo phages that do not form a phage nucleus like PA5Oct and represents a DNA phage and the RNA phage PP7 **(d),** targeting phages in other species like the lambda^{vir} phage that infects *E*. *coli,* **(e)** and inducing prophages in bacteria through ASO treatment **(f).** Anti-phage defense systems are depicted as shields, phage proteins that overcome bacterial defense as swords (Right) **a.** Transformation efficiency of PAO1 and the clinical isolate PaLo44 with empty plasmid (gentamicin resistance). After transformation, cells were spotted for CFU counting in a dilution series on LB plates with or without gentamicin. PaLo44 did not yield any plaques hence is genetically not tractable. PaLo44 was treated with an ASO against *chmA* or a non-targeting control, cells were infected with ΦKZ at a MOI of 0.0001 and CFU/PFU were determined after 3 h incubation. **b.** PAO1 and PaLo44 cells were treated with ASOs against the phage transcript *ΦKZ014* followed by ΦKZ infection and CFU/PFU determination. **c.** PA14 cells were treated consecutively with an ASO against *jukA* followed by infection with ΦKZ and CFU/PFU determination. **d.** PAO1 cells were treated with ASOs against the *portal* and *head* genes of the DNA jumbo phage PA5Oct (no nucleus-forming phage) followed by infection with PA5Oct and CFU/PFU determination. PAO1 cells were treated with ASOs against the replication (*rep*) and lysis (*lys*) genes of the RNA phage PP7 followed by infection with PP7 and CFU/PFU determination. ctrl., non-targeting control. **e.** *Escherichia coli* K12 cells were treated with ASOs against the replication *(rep)* and anti-termination (*anti-term*.) genes of the λ^{vir} phage (DNA phage) followed by infection with λ^{vir} and CFU/PFU determination. **f.** PAO1 cells were treated with ASOs followed by incubation for 3 h and CFU/PFU determination. ASOs induced prophages that resulted in lysis of *Pseudomonas* and plaque formation. **g.** PAO1 cells were treated with ASOs followed by incubation for 3 h and CFU/PFU determination. ASOs promoted plaque formation.

### DETAILED DESCRIPTION OF THE INVENTION

The present invention may be understood more readily by reference to the following detailed description of the invention and the examples included therein.

Thus, in a first aspect, the present invention relates to a method for assaying for the relevance of a protein of a phage for the replication of said phage in a bacterium, comprising the steps of (a) contacting said bacterium either before, concomitant with or after infection with said phage, in particular before infection with said phage, with at least one compound CPP-ASO^{p}, wherein CPP is a cell-penetrating peptide and ASO^{p} is an antisense construct that is specifically binding to an mRNA coding for said protein, and (b) evaluating at least one parameter that is a measure for said replication.

In the context of the present invention, the term "CPP" refers to a cell-penetrating peptide that has the ability to translocate the plasma membrane of a bacterium and is thus able to be taken up by said bacterium. Translocation may occur by a number of different mechanisms, including direct penetration of the membrane, endocytosis-mediated entry, and translocation through a transitory structure. CPPs may be naturally occurring peptides produced by living organisms, ii) chimeric peptides, which are modified natural proteins and iii) synthetic peptides entirely designed and synthesized in the laboratory. A systematic review of CPPs used for transporting nucleotide-based compositions into bacteria can be found in Gagat et al., 2027 and in El-Fateh et al., 2024. By combining a CPP with various molecular cargo molecules, such as an ASO^{p}as defined herein, it facilitates the delivery of such cargo molecules to the cytoplasm or an organelle of said bacterium. Said cargo molecules can be attached to the CPP either through chemical linkage via covalent bonds or through non-covalent interactions.

In the context of the present invention, the term "ASO" refers to short antisense oligonucleotides that bind specifically to the sequence of a target RNA and modulate protein expression through several different mechanisms. ASOs include unmodified short oligonucleotides having a phosphodiester backbone, which however are of low stability, and modified oligonucleotides, including phosphorothioates (PS), wherein the non-bridging oxygen of the phosphate group is replaced by a sulphur group, phosphorodiamidate morpholino oligomers (PMO), wherein the five-membered sugar moiety is substituted with a six-membered morpholine subunit, and each morpholine ring is inter-connected with phosphorodiamidate linkage, peptide nucleic acids (PNA), which are synthetic nucleic acid mimics that contain neutral N-2-aminoethyl glycine units, with nucleobases connected by a flexible methyl carbonyl linker, and locked nucleic acids (LNAs), which contain a constrained methylene bridge between 2' oxygen and 4' carbon of the ribose ring (see Dhuri et al. 2020). The superscripts p and c in ASO^{p} and ASO^{b}, as used herein, indicate that the respective ASO is directed at a phage and a bacterial protein, respectively. Ghosh et al. (2024) have published a comparative analysis of peptide-delivered ASOs.

In a particular embodiment, said bacterium is a Gram-negative bacterium, particularly a Gammaproteobacterium, particularly a Gammaproteobacterium selected from the Enterobacteriaceae, Vibrionaceae, or Pseudomonadaceae family, particularly selected from *Escherichia coli* and *Pseudomonas aeruginosa* species.

In a particular embodiment, said CPP is selected from the list of (KFF)3K, (RXR)4XB, (RFF)3R, (RXR)4, (RFR)4XB, (RFF)3R(3'), (RX)6B, pip1(D), drosocin, oncocin, TAT, BF2A, BF2A-RXR, drosocin-RXR, (KFF)3K(D), pip1, DAB, DAP, drosocin(D), (P59→W59)-Tat48-60, ANT, P12-(CH2)6, B12-SS, Bac1-15, IsCT-p, K6L2W3, KLW-L9,13-a, NLS-Gb3, Pep-1-K, SA-3, TDN, and TPk, in particular is selected from (KFF)3K and (RXR)4XB, more particularly is (RXR)4XB.

The reviews Gagat et al., 2027 and El-Fateh et al., 2024 that have already been mentioned above include further references to publications discussion the individual CPPs.

In a particular embodiment, the CPP is an arginine-rich cell-penetrating peptide. In a particular such embodiment, the CPP is (RXR)4XB, (RFF)3R, (RXR)4, (RFR)4XB, (RFF)3R(3'), or (RX)6B, wherein each R is L-arginine, each F is phenylalanine, each X is 6-aminohexanoic acid and B is β-alanine.

In a particular other embodiment, the CPP is a lysine-rich cell-penetrating peptide. In a particular such embodiment, the CPP is (KFF)3K or (KFF)3K wherein each K is L-lysine, and each F is phenylalanine.

In a particular embodiment, said phage is selected from: *Chimalliviridae,* in particular a Chimallivirus selected from ΦKZ, KTN4, EL, 201ϕ2-1, and ΦPA3, or a non-nucleus-forming jumbo phage, in particular PA5Oct, when the bacterium is *Pseudomonas aeruginosa,* or a Goslar Chimallivirus, when the bacterium is *Escherichia coli;* an enterobacteria phage λ, when the bacterium is *Escherichia coli;* and a single-stranded RNA phage, in particular a PP7 phage, when the bacterium is *Pseudomonas aeruginosa,* or a levivirus, when the bacterium is *Escherichia coli,* in particular a Levivirus selected from MS2 and Qβ.

In a particular embodiment, said phage is a phage forming an EPI vesicle and/or a phage nucleus inside the bacterium. In particular such embodiments, said phage is a Chimallivirus.

A Chimallivirus of particular interest is phage ΦKZ, which infects the major human pathogen, *Pseudomonas aeruginosa.*

ΦKZ-like jumbo phages are of great interest not only because of their potential for the treatment of recalcitrant *P. aeruginosa* infections via phage therapy (Chan et al. 2016, Cobián Güemes et al. 2023, Naknaen et al. 2024), but also because of their complex infection cycle that includes the sequential formation of membrane- and protein-bound compartments inside host cells. ΦKZ injects its genome together with a virion RNA-polymerase (vRNAP) inside an early phage infection (EPI) vesicle for immediate transcription of its genome (Armbruster et al. 2023, Antonova et al. 2024, Mozumdar et al. 2024). Subsequently, the phage nucleus is formed, in which the non-viron RNAP (nvRNAP) continues transcription, and the phage genome is replicated and loaded into attaching phage capsids (Chaikeeratisak et al. 2017a, reviewed in Prichard & Pogliano 2024). These phage-induced cellular compartments shield the 280-kB dsDNA genome of ΦKZ from host defense mechanisms such as CRISPR-Cas and restriction enzymes (Malone et al. 2019, Mendoza et al. 2020) and make genetic gene silencing approaches challenging. Formation of the phage nucleus also necessitates mRNA export for cytosolic translation and import of *de novo* synthesised phage proteins. Several conserved phage factors enable formation and organisation of this intracellular compartment, e.g. the nuclear shell protein chimallin (ChmA) (Chaikeeratisak et al. 2017, Laughlin et al. 2022, Nieweglowska et al. 2023), the PicA protein, which mediates cargo trafficking across the phage nucleus (Morgan et al. 2024, Kokontis et al. 2024), and the tubulin-like protein PhuZ, which centers the phage nucleus in the middle of the cell and is involved in intracellular trafficking of newly assembled capsids (Erb et al. 2014, Chaikeeratisak et al. 2017). Nevertheless, major gaps remain in our understanding of the key decision points in the ΦKZ infection cycle. ΦKZ has ~400 annotated protein-coding genes (Mesyanzhinov et al. 2002), but how many of them play an essential or important role in successful host take-over and the consecutive steps in the phage infection cycle remains unknown. This is largely due to a lack of genetic tools that permit the rapid and targeted inactivation of ΦKZ genes. Similar open issues remain with many other DNA or RNA phages.

In a particular other embodiment, said bacterium is a Gram-positive bacterium, particularly an actinobacterium or a firmicute, particularly selected from *Streptococcus,* Staphylococcus and Lactobacillus, particularly *Lactobacillus casei, Lactococcus bulgaricus* and *Streptococcus thermophilus.*

In a particular embodiment, said CPP is selected from the list of (KFF)3K, TAT (RXR)4XB, (RFR)4XB, ANT, K8, pip1 and pip1(D), in particular is selected from (KFF)3K and (RXR)4XB, more particularly is (KFF)3K.

In a particular embodiment, said phage is selected from phage PL-1 and phage phi FSW, more particularly is phage PL-1, when said bacterium is *Lactobacillus casei,* or wherein said phage is selected from *cos, pac,* 5093, and 987 group *Siphoviridae* family phages, when said bacterium is *Streptococcus thermophilus*

In a particular embodiment, said ASO^{p} is selected from DNA, RNA, peptide nucleic acids (PNAs), peptide-conjugated phosphorodiamidate morpholino oligomers (PPMOs), phosphorothioate (PTO)-modified DNA, 2'-methylated RNA (RNA-OMe), 2'-methoxyethylated RNA (RNA-MOE), 2'-fluorinated RNA (RNA-F), 2'-4'-bridged RNA (BNA), and 2'-4'-locked RNA (LNA), in particular is selected PNAs, PPMOs, PTO-modified DNA, RNA-Ome, RNA-MOE, RNA-F, BNA, and LNA, more particularly from PNAs and PPMOs, most particularly is a PNA. For a review of different ASOs, see Ghosh et al., 2024.

In a particular embodiment, said ASO^{p} is a PNA consisting of n nucleotides, wherein n is selected from 9, 10, 11, 12, 13, 14, and 15, in particular wherein n is selected from 9, 10, 11, 12, and 13, more particularly wherein n is selected from 10, 11, and 12, in particular wherein n is 10 or 11.

In a particular embodiment, said ASO^{p} is specific for a region of the gene comprising the start codon and/or the ribosomal binding site. An analysis of binding of ASOs to bacterial mRNA can be found in El-Fateh et al., 2024.

In a particular embodiment, said at least one parameter is selected from plaque-forming units, colony-forming units, formation of intracellular structures, early, intermediate and late phage transcripts, transcriptome and proteome signatures.

In a particular embodiment, said phage is a Chimallivirus and wherein said at least one parameter is the formation of an intracellular structure selected from an EPI vesicle and a phage nucleus (see Chaikeeratisak et al. 2017, reviewed in Prichard & Pogliano 2024).

In a particular embodiment, said step (a) is performed using one or more of the following conditions: cell density OD600 of 0.3, a pre-incubation time of up to 30 min, a pre-incubation time ending not more than 5 minutes before infection with said phage, a concentration of said ASO^{p} of at least 2 µM, in particular between 2 and 10 µM, more particularly between 4 and 6 µM, a multiplicity of infection (MOI) of 0.0001, a time of spotting after infection of 210 min; about 3 replication rounds and nearly full lysis of cells and a length of 10 to 11 nucleotides for the ASO^{p}.

In a particular embodiment, said protein is a protein having a putative role selected from a polymerase, a ribonuclease, capsid, endolysin, terminase, import proteins, and phylogenetically conserved proteins.

In a particular embodiment, in a first step, two or more ASO^{p} sequences and/or CPPs are tested for efficient uptake by said bacterium and/or efficient interference with the transcript encoding said protein.

In a particular embodiment, in a first step, off-target and toxic effects are excluded for the bacterium in a control experiment using said CPP-ASO^{p} without prior, concomitant or subsequent infection by said phage.

In a particular embodiment, in a first step, the knockdown of said protein is validated by a quantitative readout of the targeted protein or its phenotypic impact.

In a particular embodiment, in a first step, two or more ASO^{p}s against the same target are identified that cause a similar phenotype.

In a particular embodiment, in a first step, phenotypic readouts are compared among different phages and hosts to exclude off-target or secondary effects.

In a particular embodiment, in a first step, the minimal inhibitory ASO^{p} concentration is determined, where no plaque formation can be seen, followed by a step of using the ASO^{p} at optimal concentrations yielding the maximum log increase in plaque formation.

In the context of the present invention, the term "parameter that is a measure for said replication" refers to any parameter that can be observed, visualized, measured, determined, calculated or the like resulting in a value or characterization that is a measure for the state and/or degree of phage replication resulting from the experiment.

In a particular embodiment, said at least one parameter is selected plaque-forming units, colony-forming units, formation of intracellular structures, early, intermediate and late phage transcripts, transcriptome and proteome signatures.

In a particular embodiment, said phage is a Chimallivirus ΦKZ and wherein said at least one parameter is the formation of an intracellular structure selected from an EPI vesicle and a phage nucleus

In a particular embodiment, said step (a) is performed using one or more of the following conditions: culturing of said bacterium in MH medium; culturing said bacterium to a cell density OD600 of 0.3; performing a pre-incubation step with said CPP-ASO^{p} of up to 30 min, in particular, wherein the pre-incubation time does not end more than 5 minutes before infection with said phage; using a concentration of said CPP-ASO^{p} of at least 2 µM, in particular between 2 and 10 µM, more particularly between 4 and 6 µM; infecting said bacterium with said phage at a multiplicity of infection (MOI) of 0.0001; using a time of spotting after infection of 210 min; using about 3 replication rounds and nearly full lysis of cells; and using a length of 10 to 11 nucleotides for the ASO^{p}.

In a particular embodiment, said protein is a protein having a putative role selected from a polymerase, a ribonuclease, and an import protein.

In a second aspect, the present invention relates to a method for assaying for the relevance of a protein of a bacterium for the replication of a phage in said bacterium, comprising the steps of (a) contacting said bacterium either before, concomitant with or after infection with said phage with at least one compound CPP-ASO^{b}, wherein CPP is a cell-penetrating peptide and ASO^{b} is an antisense construct that is specifically binding to an mRNA coding for said protein, and (b) evaluation at least one parameter that is a measure for said replication..

The embodiments listed above for the first aspect apply to this second aspect as well *(mutatis mutandis).*

In a third aspect, the present invention relates to a method for inhibiting the formation of progeny particles of a phage from a bacterium infected with said phage, comprising the step of contacting said bacterium with at least one compound CPP-ASO^{p}, wherein CPP is a cell-penetrating peptide and ASO^{p} is an antisense construct that is specifically binding to an mRNA coding for a protein of said phage that is essential or important for phage replication, and/or at least one compound CPP-ASO^{b}, wherein ASO^{b} is an antisense construct that is specifically binding to an mRNA coding for a protein of said bacterium that is essential or important for phage replication.

The embodiments listed above for the first aspect apply to this third aspect as well *(mutatis mutandis).*

In a fourth aspect, the present invention relates to a method for preventing the formation of phage progeny particles from a bacterium at risk of being infected with said phage, comprising the step of contacting said bacterium with at least one compound CPP-ASO^{p}, wherein CPP is a cell-penetrating peptide and ASO^{p} is an antisense construct that is specifically binding to an mRNA coding for a protein of said phage that is essential or important for phage replication, and/or at least one compound CPP- ASO^{b}, wherein ASO^{b} is an antisense construct that is specifically binding to an mRNA coding for a protein of said bacterium that is essential or important for phage replication.

The embodiments listed above for the first aspect apply to this fourth aspect as well *(mutatis mutandis).*

In a particular embodiment, said bacterium is *Pseudomonas aeruginosa,* wherein said phage is a Chimallivirus ΦKZ and wherein said protein of said phage is selected from ΦKZ049, picA (ΦKZ069), ΦKZ042, ΦKZ155, ΦKZ056, ΦKZ174, ΦKZ177 and ΦKZ186, in particular wherein said bacterium is used in a biofilm-based fermentation (see, for example, Ren et al. 2020).

In a fifth aspect, the present invention relates to a method for promoting the formation of phage progeny particles from a bacterium comprising the steps of (a) contacting said bacterium with at least one compound CPP-ASO^{b}, wherein CPP is a cell-penetrating peptide and ASO^{b} is an antisense construct that is specifically binding to an mRNA coding for a protein essential or important for said bacterium's defence system directed against replication of said phage in said bacterium; and (b) infecting said bacterium with said phage.

The embodiments listed above for the first aspect apply to this fifth aspect as well *(mutatis mutandis).*

In a particular embodiment, said bacterium is *Pseudomonas aeruginosa,* wherein said phage is a Chimallivirus ΦKZ and wherein said protein is JukA and/or JukB.

In a sixth aspect, the present invention relates to a method for promoting the formation of phage progeny particles from a bacterium infected with, or otherwise comprising, a phage, in particular a prophage, comprising the steps of (a) contacting said bacterium with at least one compound CPP-ASO^{p/b}, wherein CPP is a cell-penetrating peptide and ASO^{p/b} is an antisense construct that is specifically binding to an mRNA coding for a phage or bacterial protein, respectively, repressing, preventing or delaying replication of said phage in said bacterium.

The embodiments listed above for the first aspect apply to this fifth aspect as well *(mutatis mutandis).*

In a particular embodiment, said step (a) results in lysis of said bacterium.

In a seventh aspect, the present invention relates to a compound CPP-ASO^{p}, wherein CPP is a cell-penetrating peptide and ASO^{p} is an antisense construct that is specifically binding to an mRNA coding for a protein repressing, preventing or delaying replication of a phage, in particular a lytic phage, in a bacterium for use in the treatment of a patient suffering from an infection with said bacterium.

The embodiments listed above for the first aspect apply to this fifth aspect as well *(mutatis mutandis).*

Birkholz et al. (2024) have shown that deletion of gene 210 (which is analogous to a knock-down with ASOs as taught in the present application) of phage ΦPA3 results in a fitness recovery of ΦKZ in a co-infection assay (see Fig. 4a,b of Birkholz et al.), since gene 210 is responsible for the inter-phage warfare and is inhibiting ΦKZ Thus, it has been shown that a gene of a first phage can have the function to inhibit a second phage. Thus, in particular variants of the second to seventh aspect, the present invention relates to a situation, where two or more different phages are present in a bacterial host cell, wherein a first phage is able to influence replication of a second phage by either inhibiting or repressing, or by starting or increasing replication of said second phage, and wherein said ASO^{p} is an antisense construct that is specifically binding to an mRNA coding for a protein of said first phage that is essential or important for replication of said second phage.

### EXAMPLES

Targeted mapping and characterization of essential or important phage genes is key to understanding the major molecular players in a phage's infection cycle and its ability to counter host defence but is restricted to few model phage-host systems that are amenable to genetic manipulation. The present approach aimed at developing a broadly applicable non-genetic route to assess gene essentiality, importance and function in phage-host interactions. Specifically, a programmable gene silencing at the RNA level was employed via exogenous delivery of synthetic antisense oligomers (ASOs) into the bacterial cytosol **(****Fig. 1a****).** Such ASOs are typically 9-12 nucleobases in length and designed to sequester the ribosome binding site (RBS) or start codon (AUG) of a target mRNA to prevent synthesis of the encoded protein. They have been applied successfully in multiple bacterial species (Nielsen 2010, Vogel 2020, Pifer & Greenberg 2020). One popular ASO modality is peptide nucleic acid (PNA), which harbours a pseudo-peptide backbone that links the four natural nucleobases and protects the oligomer from nucleolytic and proteolytic degradation. For delivery across the bacterial envelope, the ASO is fused to a cell-penetrating peptide (CPP, reviewed in El-Fateh et al. 2024). Here, we pioneer the application of ASO technology to score phage gene essentiality or importance and to understand and break defense and counter-defense mechanisms in the phage-host interplay. Seeking to identify phage genes that are required during *P. aeruginosa* infection, we perform a systematic knockdown of ΦKZ genes coupled with phenotypic and transcriptome analyses. We discover 25 essential or important genes with varying phenotypes, including a conserved phage ribonuclease that acts at a key decision point during progression from the early to the intermediate phase of the phage replication cycle. Our results suggest that more ΦKZ proteins than previously appreciated contribute to the subcellular organisation of the phage replication cycle.

Surprisingly, the present inventors have found that the ASO technology can successfully be applied to phage-infected cells and that annotated phage proteins can be silenced to investigate phage biology in bacterial host cells, in particular jumbo phage biology in diverse *Pseudomonas* strains.

Thus, to overcome the barrier, mentioned above, a non-genetic approach using exogenous delivery of synthetic programmable antisense oligomers (ASOs) to silence essential or important genes in both DNA and RNA phages of *Pseudomonas aeruginosa,* a lead pathogen in phage therapy, and *Escherichia coli.* Systematic knockdown of core and accessory genes, followed by global RNA-sequencing and microscopy analyses, led to the discovery of a set of important phage genes that can be used as targets for inhibition of phage replication. Our ASO approach promises to be widely applicable in phage biology, will help elucidate defence and anti-defence mechanisms in non-model phage-host pairs, works in clinical isolates of pathogenic bacteria, and offers a non-GMO solution for production strains for phage therapy and industrial applications.

### Example 1: Silencing of phage transcripts by ASOs

Like other bacterial species, *P. aeruginosa* is amenable to ASO-induced gene silencing, as demonstrated by bactericidal ASOs that target mRNAs encoding for essential or important proteins in bacterial transcription or fatty acid synthesis (Howard et al. 2017, El-Fateh et al. 2024). Since phage mRNAs are also translated in the bacterial cytosol, we reasoned that they should be targetable by ASOs, too **(****Fig. 1a****),** but it remained elusive if ASOs can target the rapid overloading of transcription-translation in phage-infected cells and how the handover at the phage nucleus in ΦKZ will impact ASO targeting. To establish a proof-of-concept, we designed ASOs that target the transcript of the essential chimallin protein ChmA (ΦKZ054), which is the main constituent of the phage nucleus (Chaikeeratisak et al. 2017). As a control, we treated the cells with an ASO that does not target any specific gene ('non-targeting control'). Initially, we monitored ChmA protein levels after ASO treatment using a polyclonal antibody raised against purified ChmA. 20 min post infection (p.i.), i.e. within the first round of replication, ASO knockdown strongly repressed ChmA protein levels, even at a high multiplicity of infection (MOI) of 10 (Fig. 1b).

ASOs were designed against RBS and AUG regions using the MASON algorithm (Jung et al. 2023, https://mason.helmholtz-hiri.de) and the NCBI sequence and annotation files (ΦKZ: NC_004629.1, PP7: NC_001628.1, PA14: CP000438.1, lambda: NC_001416.1, PA5Oct: NC_071039.1). ASO length was set to 10-mers for *E.c*. and 11-mers for *P*.a. and the allowed mismatches for off-targets were set to 4. ASOs were selected based on the following scoring values: melting temperature (45-55°C), low purine percentage (25-35%) and few predicted off-targets in distinct translation initiation regions of the phage (<3). The scoring values for the used ASOs are given in Supplementary Data Table 2. At least two ASOs were designed for each targeted gene. The control ASO-sequence was GACATAATTGT (ctrl.). ASOs were commercially ordered at Peps 4LS (Heidelberg) with a peptide-backbone (PNA) and a 5'-RXR ((RXRRXRRXRRXRXB)-CPP = (RXR)4XB-CPP) for *P.a*. and 5'-(KFF)₃K (KFFKFFKFFK) for *E.c*.. The initial concentration was adjusted to 1 mM in water based on the specific extinction coefficient using absorption. ASOs were stored at -20°C. Prior usage, ASOs were thawed at room temperature, then heated for 5 min at 50°C and then cooled down at room-temperature.

To test the functional effects of ChmA knockdown on phage replication, we assessed cell survival and phage progeny after multiple rounds of replication. This experimental set-up allowed us to amplify the effects on phage progeny, sample delayed replication times, and score effects that take action in the next infection round. Initially, we optimised the experimental conditions and adjusted the cell density (OD600 of 0.3), the ASO pre-treatment time (30 min), the concentration of ASOs (4-6 µM), the MOI (0.0001 to allow three rounds of replication until nearly complete host lysis), the time of spotting after infection (180 min; ~ 3 replication rounds), and the length of the ASOs (10-11 nucleobases) **(****Figure 2****).** In detail, PAO1 (DSMZ: DSM22644) was grown in LB media overnight at 37 °C and 220 rpm. Cells were inoculated 1:100 and grown in MH media at 37 °C and 220 rpm to an OD of 0.3. ASOs were added at 6 µM final concentration to 50 µl cultures and incubated for 30 min. Cells were infected with ΦKZ at an MOI 0.0001 and the cells were incubated for 3 h. 5 µl of cell culture were diluted in series and spotted on LB plates and on 0.5% LB soft agar plates with the susceptible strain PAO1 (one volume of 0.5% LB soft agar at 42 °C, was mixed with 0.01 volume cells at OD 0.5, and poured into a plate). Plates were imaged with the Typhoon 7000 phosphoimager (GE Healthcare) in fluorescence mode.

Strikingly, knockdown of ChmA caused a strong reduction in phage progeny leading to a complete loss of plaques **(Fig. 1c**), demonstrating that sterilisation can be achieved with a chmA-targeting ASO.

Overall, these results demonstrate that ASO technology can be used to inhibit essential or important phage genes with subsequent effects on phage replication.

### Example 2: Imaging of structural phenotypes and sequencing of molecular phenotypes after ASO-based gene silencing

Next, we sought to image phenotypic consequences of ChmA loss upon ASO knockdown. We therefore monitored progression through the phage replication cycle by imaging the formation of phage-derived cellular compartments. At 5-min p.i., the EPI vesicle was visible in both control and anti-*chmA* ASO treated cells **(****Fig. 2a****),** indicating successful infection. At 30-min p.i., the phage nucleus had formed in the control sample, evident as a central DNA-containing structure. ASO-based knockdown of ChmA led to formation of smaller pleiotropic cellular structures that contain DNA. We interpret these as multiple EPI vesicles due to the high MOI of 10, which is required for synchronised infection in this experimental setting. Hence, in the absence of ChmA, there is no formation of the phage nucleus, likely leading to an arrest in the phage replication cycle **(****Fig. 2a****).** This agrees with observations made in *E*. *coli* infected with the phage Goslar after silencing of ChmA via CRISPR interference by antisense RNA targeting (CRISPRi-ART, Adler et al. 2023, Armbruster et al. 2023, Morgan et al. 2024). The fact that ASO-based knockdown of ChmA completely prevented plaque formation (**Fig. 1c** )implies that the EPI vesicle is not able to support phage replication. These observations demonstrate that ASO-based silencing of phage genes is effective and can be coupled to imaging of cellular structures that form throughout the phage replication cycle to study phage biology.

Phage infection is a fine-tuned process that affects different cellular pathways, but not all will result in a macroscopic phenotype. We reasoned that more sensitive, global methods such as RNA-seq could reveal 'molecular phenotypes' after ASO-mediated phage gene knockdown, defined as specific transcriptional dysregulation in infected cells (Barquist et al. 2016, Putzeys et al. 2024).

To establish this approach for ΦKZ, we first performed high-resolution RNA-seq after ChmA knockdown, which arrests the phage replication cycle at the level of the EPI vesicle (**Fig. 2a****,** Armbruster et al. 2023). Samples were taken at 10, 15, 20, 25, 35 min after ΦKZ-infection. In the non-targeting ASO control samples a trajectory in the principal component analysis revealed the transcriptional response throughout the phage replication cycle **(****Fig. 2b****).** Under these conditions, 10 min p.i. phage transcripts represented about 45% of all sequenced reads that map to coding sequences and this increased to about 70% at 35 min p.i. (in agreement with Gerovac et al. 2024, Danilova et al. 2020, Ceyssens et al. 2017). Interestingly, the chmA transcript itself was unaffected by the ASO treatment, suggesting that in this case translational inhibition does not lead to increased mRNA depletion, as previously observed for some bacterial mRNAs targeted with ASOs (**Fig. 2c****,** Popella et al. 2022). Upon ChmA knockdown, intermediately expressed phage genes were strongly depleted from 15 min p.i. onward, indicating that formation of the phage nucleus is required for expression of these genes by the nvRNAP **(****Fig. 2c****).** Consistent with their dependence on the nvRNAP, phage tRNAs were not detected after ChmA knockdown either. Several other transcripts showed prolonged expression after ChmA knockdown, indicating potential feedback inhibition from the phage nucleus.

On the host side, under control conditions, we did not observe a substantial transcriptional response at later stages of infection compared to 10 min p.i., when host-takeover is considered to be completed. To test if this lack of a late host response is facilitated by the phage nucleus, we investigated host transcriptional changes upon ChmA knockdown. ChmA silencing caused the upregulation of 13 host transcripts out of ~3,637 detected transcripts in *P. aeruginosa* at 35 min p.i.. Among these genes were PA0201, which encodes a hypothetical hydrolase; the membrane protein FxsA, which is linked to phage exclusion and may prevent superinfection (Cheng et al. 2004, Bondy-Denomy et al. 2016); and the type III secretion system regulator SuhB, which is important for Pseudomonas virulence (Li et al. 2013). Hence, even in the absence of a phage nucleus, the host response to the EPI vesicle is limited, which proves the protective nature of this structure.

### Example 3: ASO screen for factors that are important for phage replication

The programmable feature of ASOs allows for genome-wide screens for gene essentiality or importance to discover genes critical for phage replication. ΦKZ possesses a large genome with 377 annotated genes (NCBI), of which 85% have no predicted function (Mesyanzhinov et al. 2002, De Smet et al. 2017). This paucity of functional knowledge extends also to the much smaller core genome of *Chimalliviridae,* which consists of seven blocks of genes with likely interlinked functions, plus five independent genes (Prichard et al. 2023). To identify genes that are essential or important for ΦKZ replication in *P. aeruginosa* PAO1, we screened 75 core and annotated genes (omitting most of the structural genes) using ASO-based knockdown and CFU/PFU readout.

Overall, ASO-mediated knock down of one third of these genes (25) led to a substantial effect on phage replication in CFU/PFU assays. As expected, several ASOs that target factors known to be essential or important for phage replication, such as chmA (ΦKZ054), the nvRNAP subunits (ΦKZ055 and -068) and the major head protein (ΦKZ120, **Fig. 4a**) triggered strong effects. Silencing of PicA (ΦKZ069), which is required for protein import into the phage nucleus (Morgan et al. 2024, Kokontis et al. 2024) abrogated plaque formation as well. We also observed strong effects upon knock-down of uncharacterised genes. For example, silencing of ΦKZ049, a gene that encodes a SH3 domain protein suggested to be associated with the phage DNA polymerase (lyer et al. 2021) reduced plaque formation substantially. Similarly, knock-down of ΦKZ042, which encodes a conserved but uncharacterised protein, the hypothetical proteins ΦKZ174/-177, and the early expressed transcript ΦKZ186 caused strongly reduced plaque-counts. Targeting of other genes caused milder effects (reduced plaque formation in the 1-2 order of magnitude range), although the recovery on the level of CFUs was more pronounced. An example is ΦKZ144, the gene encoding endolysin, which is vital for the final release of phage progeny from the cell (Briers et al. 2007).

In addition to CFU/PFU quantitation, we used ChmA expression as an additional readout, quantifying ChmA protein levels by immunoblotting after three rounds of replication. We observed reduced levels of ChmA for an additional 11 of the 61 genes that we screened using this read-out, even though we did not observe a strong effect on CFU/PFU. Examples include a putative RAD2/SF2 helicase (ΦKZ075), a predicted DEAD/DEAH box helicase (ΦKZ203), the macro domain-containing protein ΦKZ104, as well as the uncharacterised core genes ΦKZ176, -161, -153, and -147, and the non-core genes ΦKZ283, -286, and 056.1. Therefore, these genes have an impact on ChmA production and thus are likely to affect the phage replication cycle, although they are not essential or important for phage replication.

.Unexpectedly, there are some core genes, where ASO knockdown had no effect, e.g. see **Fig. 4a****,** ΦKZ118, -181, -188, -032, -042, -062, -070, -140.

We also observed indirect and potential off-target effects upon ASO treatment. For example, some ASOs were toxic to *P. aeruginosa* (22/176 tested ASOs overall). Other ASOs increased PFUs by one order of magnitude, indicating that the targeted proteins, such as ΦKZ151 and -306, are negative regulators of phage replication or that possible off-target effects on the host may be beneficial for phage replication. We also observed 4 ASOs that caused phage plaques without phage infection. This is indicative of activation of a prophage, most likely the integrative filamentous phage Pf4 (Knezevic et al. 2015, Gavric & Knezevic 2022).

In summary, our screening approach allowed us to identify 45 phage protein knockdowns that caused varying effects on phage replication, which can now be studied further by coupling ASO-based knock-down with phenotypic readouts or multi-omics.

After we established RNA sequencing upon knockdown of ChmA with ASOs **(****Fig. 3b,c**) and we observed for many targeted phage genes a reduction in PFUs **(****Fig. 4a****),** we reasoned that transcriptomics may be a sensitive readout that should allow the discovery of potential functional links between phage factors based on correlated transcriptomic profiles after their knockdown. We therefore extended the RNA-seq analysis to 58 additional genes from our ASO screen. At 15 min p.i., we observed diverse effects on phage transcript levels of genomic islands that did not cluster clearly. At 30 min p.i., knockdown of ChmA, the nvRNAP subunit ΦKZ055, the nvRNAP sigma factor ΦKZ068, PicA (ΦKZ069) and ΦKZ056.1, -122, -124, and -151 caused a strong effect on phage transcription **(****Fig. 4b****).** Remarkably, ChmA, the nvRNAP and PicA clearly stood out and clustered together based on ΦKZ transcript level alterations. The fact that knockdown of PicA caused the same transcriptional phenotype as knockdown of ChmA and nvRNAP suggests that the nuclear import of nvRNAP subunits might be dependent on the recently discovered PicA protein importer (Morgan et al. 2024, Kokontis et al. 2024).

On the host transcriptome, we observed diverse responses upon knockdown of phage proteins. For example, knockdown of ΦKZ174,which has a strong effect on phage replication, caused a strong downregulation of ~300 PAO1 transcripts. Curiously, knockdown of ΦKZ073 and -082 induced transcription of the Pf4 prophage locus, although it had no strong effects on PAO1 transcripts in general, suggesting a specific inhibitory mechanism of ΦKZ against the prophage. In summary, RNA sequencing revealed distinct transcriptional profiles upon knockdown of ΦKZ genes and allowed to cluster ΦKZ factors **(****Fig. 4c****)** suggesting about a hand-full of perturbation modes that allows now to illuminate their interplay in followup studies.

The CFU/PFU-based readout allowed us to sample effects arising from lack of phage progeny, replication time delays, and effects on the next round of infection. Our screen revealed that nearly one third of the targeted genes have strong effects on phage replication. These data represent rich resources of interesting phage genes for further in-depth study and a source for effective ASO targets, e.g. for the targeting of phages in productions. Our non-genetic approach is versatile and applicable to diverse phages and hosts. Coupled to phenotypic readouts such as microscopy and global transcriptome analysis, it allows functional studies of phage biology.

### Example 5: ASO-based screening identified an essential or important phage replication factor

The major goal of our ASO-based knockdown screen was to identify ΦKZ factors with roles in the progression of the phage replication cycle. Curiously, we noticed that the knockdown of ΦKZ120, -155, -177 strongly reduced plaque formation, but had very little effects on phage transcription within the time course of our analysis (Fig. 4a,b). This can be rationalised for ΦKZ120, the major head protein, which is important during later stages of infection. The same is likely true for ΦKZ177, which is also expressed very late in the replication cycle. By contrast, ΦKZ155 is expressed at 20 min p.i. and its expression is dependent on ChmA. This suggests that ΦKZ155 plays an important role after the initial formation of the phage nucleus.

To further investigate the role of ΦKZ155 during phage infection, we performed fluorescence microscopy imaging of phage infected cells. To our surprise, despite ongoing phage nucleus-dependent transcription (Fig. 4b,c), knockdown of ΦKZ155 inhibited the formation of a large phage nucleus centred within the cell at 35 min p.i. **(****Fig. 5a****).**

ΦKZ155 is part of the core genome (Prichard et al. 2023). It is predicted by sequence homology to be contain an RNase HI domain (UniProt, Pfam domain PF00075) and appears to have an uncharacterised C-terminal part. RNase HI domains are typically found in enzymes that recognize RNA-DNA heteroduplexes and specifically cleave the RNA strand (Moelling et al. 2017). To validate its nucleolytic mode of action, we produced ΦKZ155 in an *in vitro* translation (IVT) reaction and added RNA and RNA-DNA substrates. While single stranded RNA was not processed, RNA in an RNA-DNA duplex was degraded **(****Fig. 5b****).** RNA degradation was based on the RNase HI catalytic mechanism, since exchange of the conserved RNase HI catalytic residue Asp102 to Asn abrogated the nucleolytic activity of the enzyme. These data establish that ΦKZ155 has RNase Hl-like activity that does not appear to require a specific sequence motif.

RNases H-type nucleases are primarily known as enzymes that maintain DNA integrity (Moelling et al. 2017). Yet, they also degrade RNA primers that initiate genome replication in both prokaryotes and eukaryotes. Therefore, we hypothesised that ΦKZ155 might facilitate ΦKZ genome amplification. Indeed, after ASO-mediated silencing of ΦKZ155, we observed no phage genome amplification **(****Fig. 5c****).** Of note, the lack of ΦKZ155 causes an arrest in phage nucleus maturation that allows to illuminate this transient step in follow-up studies **(****Fig. 5a****).** Here, we applied ASO-based knockdowns coupled to quantitative readouts of well described features in the phage replication cycle to pinpoint the moment of action of ΦKZ155 that apparently plays a role shortly after the initial phage nucleus is made and facilitates a handover to the maturation of the phage nucleus and represents a novel phage replication factor that can be targeted by ASOs to prevent phage replication.

### Example 6: Silencing of phage genes in genetically not tractable clinical isolates

To prove the versatility of ASOs in the study of phage-host interactions, we extended the approach to genetically intractable strains, targeting either phage or host genes. *P. aeruginosa* strain PaLo44 is a clinical isolate (NCBI, PRJNA731114) that like many other strains isolated from patient samples has been refractory to transformation using classical approaches of bacterial genetics, such as electroporation **(****Fig. 6a****).** Therefore, these strains are not amenable to CRISPR-based approaches. Using ASOs to target *chmA,* we were able to inhibit ΦKZ replication in PaLo44 with the same efficiency as observed in the *P. aeruginosa* lab strain PAO1 used above **(****Fig. 6a****).**

### Example 7: Silencing of phage genes that target anti-phage defence systems to allow for phage replication.

The clinical isolate PaLo44 unlike PAO1- is not susceptible to infection with a ΦKZ phage lacking the ΦKZ014 gene, which encodes a ribosome-associated protein that appears to circumvent a PaLo44-specific defense system (Gerovac et al. 2024). Using ASO-based knockdown of ΦKZ014 **(****Fig. 6b****),** we were able to render PaLo44 resistant to ΦKZ as previously observed with an engineered ΔΦKZ014 phage (Gerovac et al. 2024). These data show that ASOs can be used to screen the impact of phage factors in diverse hosts without the need of genetic engineering.

### Example 8: Silencing of anti-phage defence systems in the host to render pathogens susceptible for phage infections.

Host-encoded defence systems are a major barrier to phage replication, but their genomic disruption is challenging. ASOs could be an alternative approach to inhibit their expression. To test this, we selected the JukAB defence system in the P. aeruginosa strain PA14, which rescues the cell by preventing early phage transcription, DNA replication, and nucleus assembly (Li et al. 2022). Since the jukAB locus is constitutively active, we inhibited the production of jukA prior to phage infection via repeated addition of a complementary ASO. ASO-mediated silencing of the jukA mRNA rendered *P. aeruginosa* PA14 bacteria susceptible to ΦKZ and allowed phage replication **(****Fig. 6c****),** indicating successful knockdown of the defence system. These results demonstrate that ASOs can be used to inhibit phage defence systems to study their composition and sensitivity in their native host and to propagate phages in otherwise non-permissive (pathogenic) strains.

### Example 9: Silencing phage genes in other DNA and in RNA phages

Other jumbo phages do not form a phage nucleus and are very different in their replication cycle, for example, PA5Oct that also infects PAO1. We established ASO-based knockdowns also here against the portal and head protein transcripts that completely abrogated plaque formation **(****Fig. 6d****).**

RNA phages are an understudied class of bacterial viruses (Callanan et al. 2018), although knowledge of their diversity is expanding owing to novel techniques that reduce the sampling bias that normally favours detection of DNA phages (Neri et al. 2022). To test the applicability of ASO technology to RNA phages, we selected the model RNA phage PP7, which also infects P. aeruginosa PAO1. We targeted the replication (rep, RNA-dependent RNA polymerase, RdRP) and lysis (*lys*) genes of PP7 with ASOs and completely abrogated plaque formation **(****Fig. 6d****).** These data demonstrate that ASO-based knockdown of essential or important phage genes is applicable to DNA and RNA phages.

### Example 10: Silencing phage genes in phylogenetically distant species like E. coli

We focused on the lytic model phage λ^{vir}, which infects *E*. *coli* and designed ASOs that target the transcripts of five essential or important genes (O for replication (*rep*), Q anti-termination factor (anti-term.)). Since we previously established that KFF is able to deliver ASOs in *E*. *coli* (Popella et al. 2021, Popella et al. 2022), we choose KFF as carrier peptide and 10-mer ASOs. After pre-incubation of cells at OD600 0.03 with the different ASOs (10 µM, single dose), we infected cells with the phage and assessed bacterial colonies (colony forming units, CFU) and phage plaques (plaque forming units, PFU) after three rounds of replication to amplify the effect and to cover effects that become apparent only upon re-infection **(****Fig. 6e****).** Knockdown of essential or important phage genes in λ^{vir} yielded a reduction of plaques by 2-3 magnitudes and recovered bacterial growth at lower dilutions. This effect was in a similar range as previously described in a genome-wide CRISPRi approach targeting the same genes (Piya et al. 2023), indicating efficient ASO-mediated knockdown of the target genes.

### Example 11: Induction of prophages in bacteria with ASO treatment

We also observed indirect and potential off-target effects upon ASO treatment. For example, some ASOs were toxic to P. aeruginosa (22/176 tested ASOs overall). We also observed 4 ASOs that caused phage plaques without phage infection **(****Fig. 6f****).** This is indicative of activation of a prophage, most likely the integrative filamentous phage Pf4 (Knezevic et al. 2015, Gavric & Knezevic 2022).

### Example 12: Promotion of phage replication with ASO treatment

In other cases, we observed that ASO-based targeting of phage factors yielded more plaques indicating that ASOs can be used to promote the replication of phages **(****Fig. 6g****).**

### Discussion

Here we demonstrate that ASO technology is effective in silencing phage and host transcripts to investigate biology at the host-phage interface. We applied this approach to screen core and functionally annotated genes of the jumbo phage ΦKZ for their essentiality or importance and their effect on the phage and host transcriptomes upon infection. The CFU/PFU-based readout allowed us to sample effects arising from lack of phage progeny, replication time delays, and effects on the next round of infection. Our screen revealed that nearly one third of the targeted genes have strong effects on phage replication. These data represent rich resources of interesting phage genes for further in-depth study and a source for effective ASO targets, e.g. for the targeting of phages in productions. Our non-genetic approach is versatile and applicable to diverse phages and hosts. Coupled to phenotypic readouts such as microscopy and global transcriptome analysis, it allows functional studies of phage biology. Through this approach, we identified the RNase Hl-domain containing protein ΦKZ155 as an essential factor linked to genome amplification and a novel target for ASO-based knockdown of phage replication.

ASOs can be used in versatile ways to study the phage-host conflict. For example, knockdown of anti-phage defence systems, shown here for JukAB **(****Fig. 6c****).** Of note, the effectors of anti-phage defence systems can be activated also by a cellular response that can be also a general stress response and is dependent on the strain's physiological state and stress tolerance. ASO-based knockdowns allow the study of defense responses in the native host that can be extended to screens of strain libraries that are often genetically intractable. This is particularly important in clinical isolates that are genetically not tractable **(****Fig. 6a****).** Directing ASO-based knockdowns against the host is also relevant in the context of phage therapy. For example, knockdown of anti-phage defence systems can be used to render pathogens susceptible to phages **(****Fig. 6c****)** or to promote phage replication **(****Fig. 6g****)** by co-application of ASOs and phage cocktails, e.g. in wound infections. Alternatively, reducing the expression levels of anti-phage defence systems in clinical isolates through ASO treatment might allow initial infection by model phages, which could then evolve through escaper mutations into novel therapeutic phages. Similarly, ASOs could be used to optimise production strains for therapeutic phages. Of note, we observed that some ASOs cause prophage induction **(****Fig. 6f****),** which could be used as a route to target self-lysis of pathogens in therapy. On the other hand, ASO-based knockdown of phage genes can also be used to render phages compatible with other phages in phage cocktails. For example, ASOs could be used in phage co-infection assays to identify phage factors that are involved in inter-phage warfare (Birkholz et al. 2024b), potentially leading to novel phage combinations in phage therapy with ASOs that could be also used to adjusted phage cocktail therapy throughout the treatment.

ASOs could be used as a virucide for sterilisation through inhibition of well-characterised essential phage transcripts, e.g. the phage polymerase and capsid. This was shown here for the jumbo phages ΦKZ that forms a phage nucleus and PA5Oct that does not form a phage nucleus **(****Fig. 6d****),** for the RNA phage PP7 **(****Fig. 6d****),** and for the *E*. *coli* phage Lambda^{Vir} **(****Fig. 6e****)** that shows applicability of ASOs in phylogenetically distant species. In addition, our screen revealed six other uncharacterised targets (**Fig. 4a,c**) that lend itself for targeting of nucleus-forming phages by ASOs. Importantly, ASO-based knockdown does not require genetic engineering of cells. Therefore, this approach does not require permissions to work with genetically modified organisms (GMO). It represents a non-genetic approach that is applicable to diverse phages and hosts and may accelerate phage research and therapy.

### Methods

**ASOs.** ASOs were designed against RBS and AUG regions using the MASON algorithm (Jung et al. 2023, https://mason.helmholtz-hiri.de) and the NCBI sequence and annotation files (ΦKZ: NC_004629.1, PP7: NC_001628.1, PA14: CP000438.1, λ^{vir}: NC_001416.1, PA5Oct: NC_071039.1). ASO length was set to 10-mers for *E.c*. and 11-mers for *P.a*. and the allowed mismatches for off-targets were set to 4. ASOs were selected based on the following scoring values: melting temperature (45-55°C), low purine percentage (25-35%) and few predicted off-targets in distinct translation initiation regions of the phage (<3). At least two ASOs were designed for each targeted gene. The control ASO-sequence was GACATAATTGT (ctrl.). ASOs were commercially ordered at Peps 4LS (Heidelberg) with a peptide-backbone (PNA) and a 5'-RXR (RXRRXRRXRRXRXB) CPP for *P.a*. and 5'-(KFF)₃K (KFFKFFKFFK) for *E.c.*. The initial concentration was adjusted to 1 mM in water based on the specific extinction coefficient using absorption. ASOs were stored at -20°C. Prior usage, ASOs were thawed at room temperature, then heated for 5 min at 50°C and then cooled down at room-temperature.

**CFU/PFU assay.** PAO1 (JVS-11761, DSMZ: DSM22644), PaLo44 (R. Lavigne lab, KU Leuven, Belgium), PA14 (R. Lavigne lab, KU Leuven, Belgium) were grown in LB media overnight at 37°C and 220 rpm. Cells were inoculated 1:100 and grown in MH media at 37°C and 220 rpm to an OD of 0.3. ASOs were added at 6 µM final concentration to 50 µl cultures and incubated for 30 min. Cells were infected with ΦKZ at an MOI 0.0001 and the cells were incubated for 3 h. 5 µl of cell culture were diluted in series and spotted on LB plates and on 0.5% LB soft agar plates with the susceptible strain PAO1 (one volume of 0.5% LB soft agar at 42 °C, was mixed with 0.01 volume cells at OD 0.5, and poured into a plate). Plates were imaged with the Typhoon 7000 phosphor imager (GE Healthcare) in fluorescence mode.

For PA14, the cells were inoculated 1:100 in MH media with 8 µM final concentration ASOs and every hour 8 µM final concentration ASOs were added additionally until the cells were harvested for CFU/PFU analysis after 3 h.

For PP7 infection, PAO1 cells were inoculated 1:100 in MH media and treated at OD 0.3 with 0.05 µM final concentration ASOs, additionally every 30 min 0.05 µM final concentration ASOs were added. Cells were infected 30 min after starting treatment with PP7 at an MOI of 0.00001, and cells were harvested for CFU/PFU analysis after 3.5 h.

**Immunoblotting.** Infected cell cultures were mixed with final 1× SDS-PAGE loading dye (60 mM Tris/HCl pH 6.8, 0.2 g/ml sodium dodecyl sulphate (SDS), 0.1 mg/ml bromophenol blue, 77 mg/ml DTT, 10% (v/v) glycerol) and were boiled for 10 min at 95 °C for denaturation. Protein samples were analysed by SDS-PAGE and blotted onto methanol-preactivated polyvinylidene (PVDF) membranes. As a loading control, we used Coomassie staining of a second gel where we loaded the same sample volume. ChmA was produced as previously described (Laughlin et al. 2022) in *E. coli* BL21-CodonPlus (DE3)-RIL cells (Agilent Technologies, JVS-12280, chloramphenicol resistance) that were transformed with pET-M14(+) plasmid carrying the *chmA* gene with a N-terminal His-V5-TEV-tag (pMiG118). The tag could not be removed in the purification procedure. Commercial antibody sera were generated at Eurogentec. Rabbits were immunised with the purified protein. The rabbit serum (no. 2481) was used together with anti-rabbit-HRP antibody (Thermo Scientific, 31460) in 5% BSA/TBST for ChmA detection in immunoblotting. Antibody specificity was validated in immunoblotting by comparison between ΦKZ-infected and non-infected cells that yielded a defined band at 70 kDa corresponding for ChmA only in infected cells **(****Fig. 1b****).** Of note, crossreactivity was observed for the host proteome that could be depleted upon repeated use of the antibody.

**Microscopy.** 0.85% (w/v) agarose was dissolved in 5-fold water diluted LB media and boiled to melt. The liquid agarose was poured on microscope slides with one slide pair at each side as a spacer and one slide on top to form a closed gel slice as described in (Skinner et al. 2013). After solidification ~1×1 cm pads were cutted. Cells were grown in MH media at 220 rpm and 37 °C to an OD600 ~0.3, then preincubated with ASOs for 30 min, and infected with ΦKZ at an MOI of 5. At indicated time points the phage replication cycle was quenched by cooling the cells on ice for 10 min and the cells were pelleted at 8k×*g* for 5 min. The supernatant was removed and the cells were resuspended in 500 µl 4% paraformaldehyde and incubated for 15 min on ice. Afterwards cells were washed with PBS and were resuspended in 50 µl PBS for storage at 4 °C. Cells were stained with 16 µM FM4-64, and 360 nM DAPI and 5 µl were layered onto 1.2% agarose pads. The pad was placed with the side of application downwards into a µ-Slide 8 Well high Grid-500. Transmission and fluorescence were detected with a confocal laser scanning microscope Leica SP5. Images were processed with ImageJ (1.53).

For the imaging of ΦKZ155-GFP, PAO1 cells were transformed with a plasmid (pLBu005) coding for ΦKZ155-GFP under the control of an inducible pBAD promoter (arabinose inducible), and selected on gentamycin plates. The cells (JVS-13713) were grown to an OD 0.25 and induced with arabinose at indicated concentrations followed by phage infection with an MOI of 5 at OD 0.3. The harvesting, crosslinking and imaging of cells was conducted as described previously for wt cells.

**RNA preparation and sequencing.** PAO1 was grown o/n in LB at 37 °C 220 rpm. Cells were inoculated 1:100 in MH media at 37 °C and grown until OD₆₀₀ 0.3. 6 µM ASO (JVpna-79 for control or -72 for *chmA* inhibition) were added to 1.6 ml of cell culture and incubated for 30 min at 37 °C 220 rpm. Cells were infected with ΦKZ at an MOI of 5. At indicated time points, 250 µl were removed and put on ice. Infection efficiency was independently validated by confocal microscopy and CFU spotting with 50 µl of cells. RNA was isolated from 200 µl of cells using the RNAsnap procedure (Stead et al. 2012). Two volumes of RNAprotect (Qiagen) were added and cells were incubated for 5 min. Cells were pelleted at full-speed for 20 min at 4 °C and the supernatant was removed. The pellet was resuspended in 100 µl SNAP buffer (0.025% SDS, 18 mM EDTA, 1% β-mercaptoethanol, 95% formamide (RNA-grade)). Samples were incubated for 7 min at 95 °C, cell debris was pelleted at full-speed for 5 min at room-temperature, and the supernatant was transferred to a new tube. 1.5 volumes of ethanol was added to the supernatant and the sample was mixed by pipetting. The sample was loaded onto a miRNeasy mini column (Qiagen) two times and spun at full-speed for 20 s at room-temperature. Columns were washed two times with 500 µl RPE buffer (Qiagen) and spun at 8k×*g* for one minute at room-temperature. One final spin was used to dry the column in an empty tube. 30 µl RNase-free water was added to the column and the RNA eluted at 8k×*g* for one minute at room-temperature. The elution was repeated with the flow-through to recover more RNA. The RNA concentration was determined by absorption at 260 nm. RNA was stored at -80 °C.

RNA-sequencing was performed at the CoreUnit SysMed at the University of Würzburg. DNA was digested with DNasel and the rRNA was depleted with the Lexogen RiboCOP META depletion kit. RNA library was prepared with the CORALL Total RNA-Seq Library Prep Kit V1 (Lexogen). The library was sequenced on the NextSeq2000 (Illumina) with a P1-seq kit (single-end 1x100 bp, Illumina). RNA-seq analysis for the ChmA knockdown and the screen was conducted with READemption 0.4.3 and 2.0.4 (Förstner et al. 2014), respectively. Reads were aligned for PAO1 and ΦKZ to NC_002516 and NC_004629, respectively. Enrichment of transcripts was calculated with the DeSeq module in READemption. Read-coverage was illustrated with the Integrated Genomics Viewer (IGV, Robinson et al. 2011).

We defined early and intermediate phage transcripts based on their significant enrichment (log₂fold >2 or log₂fold <-2, -log₁₀p_{adj} >10) between the control 35- and 10-min samples (similar as in Ceyssens et al. 2014).

***In vitro* translation.** Template DNA was produced via PCR and Taq-polymerase followed by gel purification. JVO-23244 and -5 were used to amplify wt *ΦKZ155* with a T7 promoter from ΦKZ lysate, and for *ΦKZ155^{D102N}* pLBu021 was used as template. The template DNA was amplified with JVO-23244/-5 primers adding T7 promoter to the amplified fragment. 250 ng template DNA was supplemented in 10 µl PURExpress *in vitro* protein synthesis kit mix (NEB). The reaction mix was incubated for 2 h at 30 °C and was subsequently used for assays.

**Cleavage assays.** RNA, DNA templates (JVRNA-001, AUAUAAGGGAACAUAGAUAAACCCCUCCCUAAUAAAAUG, JVO-23272, ATATAAGGGAACATAGATAAACCCCTCCCTAATAAAATG) were 5'-³²P-labeled, mixed in 1:2 ratio together with the reverse complement DNA (JVO-23273, CATTTTATTAGGGAGGGGTTTATCTATGTTCCCTTATAT), boiled and slowly cooled down to room temperature in a water bath to anneal the duplexes. 1 pmol were added to 2 µl PURExpress IVT mix that translated for 2 h a control, PHIKZ155, PHIKZ155^{D102N}, or other nucleases as indicated. The mix was incubated for 1 h, mixed with one volume GLII buffer, boiled for 5 min, rapidly cooled down on ice, and loaded onto 6% 6 M Urea-PAGE (19:1) gels that were run at 300 V for 2 h. The gel was transferred onto Whatman filter paper, vacuum dried, and a phosphor image screen was used to read out the autoradiogram on a Typhoon FLA7000 imager (GE). As a positive control, we used a commercially available RNase H (NEB, M0297S).

**Dot-Southern-blotting.** PAO1 cells were grown to OD 0.3 in MH media and were pretreated with 6 µM ASOs against ΦKZ nucleases for 30 min. Cells were infected at an MOI of 10 with ΦKZ. At indicated time points a fraction of the culture was removed and 1% SDS was added followed by boiling at 95 °C for 5 min. Subsequently the DNA was extracted from the sample with PCI, and subsequently with one volume chloroform. The aqueous phase was supplemented with 1.5 volume 1 M NaOH and 15 mM EDTA (pH 9). The sample was heated for 3 min at 95 °C and put on ice for 5 min. The solution was filtered with a dot blot apparatus through an equilibrated (0.3 M NaOH) and positively charged nylon membrane. Subsequently, the membrane was dried and the DNA crosslinked via exposition to UV light for 5 min. The membrane was equilibrated with hybridization solution for two times, and a radiolabeled oligo was added for hybridization o/n starting at 60 °C for 1 h and then 48 °C overnight. The membrane was washed once for 15 min with 2×SSC, and 0.5×SSC, and a screen was used for phosphor imaging on a Typhoon FLA7000 imager (GE).

### References

Abramson, J., Adler, J., Dunger, J., Evans, R., Green, T., Pritzel, A., Ronneberger, O., Willmore, L., Ballard, A. J., Bambrick, J., Bodenstein, S. W., Evans, D. A., Hung, C.-C., O'Neill, M., Reiman, D., Tunyasuvunakool, K., Wu, Z., Žemgulytė, A., Arvaniti, E., ... Jumper, J. M. (2024). Accurate structure prediction of biomolecular interactions with AlphaFold 3. Nature, doi: 10.1038/s41586-024-07487-w.
Adler, B.A., Al-Shimary, M.J., Patel, J.R., Armbruster, E., Colognori, D., Charles, E.J., Miller, K.V., Lahiri, A., Trinidad, M., Boger, R., et al. (2023). Genome-wide characterization of diverse bacteriophages enabled by RNA-binding CRISPRi. bioRxiv, doi: 10.1101/2023.09.18.558157.
Antonova, D., Nichiporenko, A., Sobinina, M., Vishnyakov, I. E., Moiseenko, A., Kurdyumova, I., Khodorkovskii, M., Sokolova, O. S., & Yakunina, M. V. (2024). Genomic transfer via membrane vesicle: A strategy of giant phage phiKZ for early infection. bioRxiv. doi: 10.1101/2023.12.31.573766.
Armbruster, E., Lee, J., Hutchings, J., VanderWal, A., Enustun, E., Adler, B., Aindow, A., Deep, A., Rodriguez, Z., Morgan, C., et al. (2023). Sequential membrane- and protein-bound organelles compartmentalize genomes during phage infection. bioRxiv. doi: 10.1101/2023.09.20.558163.
Bernheim, A., & Sorek, R. (2020). The pan-immune system of bacteria: antiviral defence as a community resource. Nature Reviews. Microbiology, 18(2), 113-119. doi: 10.1038/s41579-019-0278-2.
Birkholz, E. A., Morgan, C. J., Laughlin, T. G., Lau, R. K., Prichard, A., Rangarajan, S., Meza, G. N., Lee, J., Armbruster, E., Suslov, S., Pogliano, K., Meyer, J. R., Villa, E., Corbett, K. D., & Pogliano, J. (2024). An intron endonuclease facilitates interference competition between coinfecting viruses. Science, 385(6704), 105-112, doi: 10.1126/science.adl1356.
Bondy-Denomy, J., Qian, J., Westra, E. R., Buckling, A., Guttman, D. S., Davidson, A. R., & Maxwell, K. L. (2016). Prophages mediate defense against phage infection through diverse mechanisms. The ISME Journal, 10(12), 2854-2866, doi: 10.1038/ismej.2016.79.
Bondy-Denomy, J., Pawluk, A., Maxwell, K. L., & Davidson, A. R. (2013). Bacteriophage genes that inactivate the CRISPR/Cas bacterial immune system. Nature, 493(7432), 429-432, doi: 10.1038/nature11723.
Bondy-Denomy, J., Qian, J., Westra, E.R., Buckling, A., Guttman, D.S., Davidson, A.R., and Maxwell, K.L. (2016). Prophages mediate defense against phage infection through diverse mechanisms. ISME J. 10, 2854-2866.
Erica A. Birkholz, E. A., Morgan, C. J., Laughlin, T. G., Lau, R. K., Prichard, A., Rangarajan, S., Meza, G. N., Lee, J., Armbruster, E., Suslov, S., Pogliano, K., Meyer, J. R., Villa, E., Corbett, K. D. & Pogliano, J. (2024). An intron endonuclease facilitates interference competition between coinfecting viruses. Science, 385, 105-112.
Briers, Y., Volckaert, G., Cornelissen, A., Lagaert, S., Michiels, C. W., Hertveldt, K., & Lavigne, R. (2007). Muralytic activity and modular structure of the endolysins of Pseudomonas aeruginosa bacteriophages phiKZ and EL. Molecular Microbiology, 65(5), 1334-1344, doi: 10.1111/j.1365-2958.2007.05870.x.
Callanan, J., Stockdale, S. R., Shkoporov, A., Draper, L. A., Ross, R. P., & Hill, C. (2018). RNA phage biology in a metagenomic era. Viruses, 10(7), doi: 10.3390/v1 0070386.
Camara-Wilpert, S., Mayo-Muñoz, D., Russel, J., Fagerlund, R. D., Madsen, J. S., Fineran, P. C., Sorensen, S. J., & Pinilla-Redondo, R. (2023). Bacteriophages suppress CRISPR-Cas immunity using RNA-based anti-CRISPRs. Nature, 623(7987), 601-607, doi: 10.1038/s41586-023-06612-5.
Ceyssens, P.-J., Minakhin, L., Van den Bossche, A., Yakunina, M., Klimuk, E., Blasdel, B., De Smet, J., Noben, J.-P., Blasi, U., Severinov, K., & Lavigne, R. (2014).
Development of giant bacteriophage ΦKZ is independent of the host transcription apparatus. Journal of Virology, 88(18), 10501-10510, doi: 10.1128/jvi.01347-14.
Chaikeeratisak, V., Nguyen, K., Khanna, K., Brilot, A.F., Erb, M.L., Coker, J.K.C., Vavilina, A., Newton, G.L., Buschauer, R., Pogliano, K., et al. (2017). Assembly of a nucleus-like structure during viral replication in bacteria. Science 355, 194-197.
Chaikeeratisak, V., Birkholz, E. A., & Pogliano, J. (2021). The phage nucleus and PhuZ spindle: defining features of the subcellular organization and speciation of nucleus-forming jumbo phages. Frontiers in Microbiology, 12, 641317, doi: 10.3389/fmicb.2021.641317.
Chan, B. K., Sistrom, M., Wertz, J. E., Kortright, K. E., Narayan, D., & Turner, P. E. (2016). Phage selection restores antibiotic sensitivity in MDR Pseudomonas aeruginosa. Scientific Reports, 6(1), 26717, doi: 10.1038/srep26717.
Cheng, X., Wang, W., & Molineux, I. J. (2004). F exclusion of bacteriophage T7 occurs at the cell membrane. Virology, 326(2), 340-352, doi: 10.1016/j.virol.2004.06.001.
Cobián Güemes, A. G., Ghatbale, P., Blanc, A. N., Morgan, C. J., Garcia, A., Leonard, J., Huang, L., Kovalick, G., Proost, M., Chiu, M., Kuo, P., Oh, J., Karthikeyan, S., Knight, R., Pogliano, J., Schooley, R. T., & Pride, D. T. (2023). Jumbo phages are active against extensively drug-resistant eyedrop-associated Pseudomonas aeruginosa infections. Antimicrobial Agents and Chemotherapy, 67(12), e0065423, doi: 10.1128/aac.00654-23.
Danilova, Y. A., Belousova, V. V., Moiseenko, A. V., Vishnyakov, I. E., Yakunina, M. V., & Sokolova, O. S. (2020). Maturation of pseudo-nucleus compartment in P. aeruginosa, infected with giant phiKZ phage. Viruses, 12(10), doi: 10.3390/v12101197.
De Smet, J., Hendrix, H., Blasdel, B. G., Danis-Wlodarczyk, K., & Lavigne, R. (2017). Pseudomonas predators: understanding and exploiting phage-host interactions. Nature Reviews Microbiology, 15(9), 517-530, doi: 10.1038/nrmicro.2017.61.
Dhuri, K., Bechtold, C., Quijano, E., Pham, H., Gupta, A., Vikram, A., & Bahal, R. (2020). Antisense oligonucleotides: An emerging area in drug discovery and development. Journal of Clinical Medicine Research, 9(6), doi: 10.3390/jcm9062004
El-Fateh, M., Chatterjee, A., & Zhao, X. (2024). A systematic review of peptide nucleic acids (PNAs) with antibacterial activities: Efficacy, potential and challenges. International Journal of Antimicrobial Agents, 63(3), 107083, doi: 10.1016/j.ijantimicag.2024.107083.
Erb, M. L., Kraemer, J. A., Coker, J. K. C., Chaikeeratisak, V., Nonejuie, P., Agard, D. A., & Pogliano, J. (2014). A bacteriophage tubulin harnesses dynamic instability to center DNA in infected cells. eLife, 3(3), e03197, doi: 10.7554/eLife.03197.
Förstner, K. U., Vogel, J., & Sharma, C. M. (2014). READemption-a tool for the computational analysis of deep-sequencing-based transcriptome data. Bioinformatics , 30(23), 3421-3423, doi: 10.1093/bioinformatics/btu533.
Gagat, M., Zielińska, W., & Alina Grzanka (2017). A. Cell-penetrating peptides and their utility in genome function modifications (Review). Int J Mol Med. 40(6), 1615-1623, doi: 10.3892/ijmm.2017.3172
Gavric, D., & Knezevic, P. (2022). Filamentous Pseudomonas phage Pf4 in the context of therapy-inducibility, infectivity, lysogenic conversion, and potential application. Viruses, 14(6), doi: 10.3390/v14061261.
Gerovac, M., Chihara, K., Wicke, L., Böttcher, B., Lavigne, R., & Vogel, J. (2024). Phage proteins target and co-opt host ribosomes immediately upon infection. Nature Microbiology, 9(3), 787-800, doi: 10.1038/s41564-024-01616-x.
Ghosh, G., Popella, L., Dhamodharan, V., Jung, J., Dietzsch, J., Barquist, L., Höbartner, C., & Vogel, J. (2024) A comparative analysis of peptide-delivered antisense antibiotics using diverse nucleotide mimics. RNA. 30(6): 624-643 doi: 10.1261 /rna.079969.124
Guan, J., Oromí-Bosch, A., Mendoza, S. D., Karambelkar, S., Berry, J. D., & Bondy-Denomy, J. (2022). Bacteriophage genome engineering with CRISPR-Cas13a. Nature Microbiology, 7(12), 1956-1966, doi: 10.1038/s41564-022-01243-4.
Hobbs, L. J., & Nossal, N. G. (1996). Either bacteriophage T4 RNase H or Escherichia coli DNA polymerase I is essential for phage replication. Journal of Bacteriology, 178(23), 6772-6777, doi: 10.1128/jb.178.23.6772-6777.1996.
Hollingsworth, H. C., & Nossal, N. G. (1991). Bacteriophage T4 encodes an RNase H which removes RNA primers made by the T4 DNA replication system in vitro. The Journal of Biological Chemistry, 266(3), 1888-1897. https://www.ncbi.nlm.nih.gov/pubmed/1703156.
Howard, J. J., Sturge, C. R., Moustafa, D. A., Daly, S. M., Marshall-Batty, K. R., Felder, C. F., Zamora, D., Yabe-Gill, M., Labandeira-Rey, M., Bailey, S. M., Wong, M., Goldberg, J. B., Geller, B. L., & Greenberg, D. E. (2017). Inhibition of Pseudomonas aeruginosa by peptide-conjugated phosphorodiamidate morpholino oligomers. Antimicrobial Agents and Chemotherapy, 61(4), doi: 10.1128/AAC.01938-16.
Itoh, T., & Tomizawa, J. (1980). Formation of an RNA primer for initiation of replication of ColE1 DNA by ribonuclease H. Proceedings of the National Academy of Sciences of the United States of America, 77(5), 2450-2454, doi: 10.1073/pnas.77.5.2450.
Iyer, L. M., Anantharaman, V., Krishnan, A., Burroughs, A. M., & Aravind, L. (2021). Jumbo phages: a comparative genomic overview of core functions and adaptions for biological conflicts. Viruses, 13(1), doi: 10.3390/v13010063.
Jung, J., Popella, L., Do, P. T., Pfau, P., Vogel, J., & Barquist, L. (2023). Design and off-target prediction for antisense oligomers targeting bacterial mRNAs with the MASON web server. RNA , 29(5), 570-583, doi: 10.1261/rna.079263.122.
Knezevic, P., Voet, M., & Lavigne, R. (2015). Prevalence of Pf1-like (pro)phage genetic elements among Pseudomonas aeruginosa isolates. Virology, 483, 64-71, doi: 10.1016/j.virol.2015.04.008.
Kokontis, C., Klein, T. A., Silas, S., & Bondy-Denomy, J. (2024). Conserved jumbo phage factors required for protein import into a phage nucleus. bioRxiv, doi: 10.1101/2024.03.27.586873.
Kole, R., Krainer, A.R., and Altman, S. (2012). RNA therapeutics: beyond RNA interference and antisense oligonucleotides. Nat. Rev. Drug Discov. 11, 125-140.
Laughlin, T. G., Deep, A., Prichard, A. M., Seitz, C., Gu, Y., Enustun, E., Suslov, S., Khanna, K., Birkholz, E. A., Armbruster, E., McCammon, J. A., Amaro, R. E., Pogliano, J., Corbett, K. D., & Villa, E. (2022). Architecture and self-assembly of the jumbo bacteriophage nuclear shell. Nature, 608(7922), 429-435, doi: 10.1038/s41586-022-05013-4.
Li Kewei, Xu Chang, Jin Yongxin, Sun Ziyu, Liu Chang, Shi Jing, Chen Gukui, Chen Ronghao, Jin Shouguang, & Wu Weihui. (2013). SuhB is a regulator of multiple virulence genes and essential for pathogenesis of Pseudomonas aeruginosa. mBio, 4(6), 10.1128/mbio.00419-13, doi: 10.1128/mbio.00419-13.
Li, Y., Guan, J., Hareendranath, S., Crawford, E., Agard, D. A., Makarova, K. S., Koonin, E. V., & Bondy-Denomy, J. (2022). A family of novel immune systems targets early infection of nucleus-forming jumbo phages. bioRxiv, 2022.09.17.508391, doi: 10.1101/2022.09.17.508391.
Malone, L. M., Warring, S. L., Jackson, S. A., Warnecke, C., Gardner, P. P., Gumy, L. F., & Fineran, P. C. (2020). A jumbo phage that forms a nucleus-like structure evades CRISPR-Cas DNA targeting but is vulnerable to type III RNA-based immunity. Nature Microbiology, 5(1), 48-55, doi: 10.1038/s41564-019-0612-5.
Marsh, J. W., Kirk, C., & Ley, R. E. (2023). Toward microbiome engineering: Expanding the repertoire of genetically tractable members of the human gut microbiome. Annual Review of Microbiology, 77, 427-449, doi: 10.1146/annurev-micro-032421-112304.
Mayo-Muñoz, D., Pinilla-Redondo, R., Birkholz, N., & Fineran, P. C. (2023). A host of armor: Prokaryotic immune strategies against mobile genetic elements. Cell Reports, 42(7), 112672, doi: 10.1016/j.celrep.2023.112672.
Mayo-Muñoz, D., Pinilla-Redondo, R., Camara-Wilpert, S., Birkholz, N., & Fineran, P. C. (2024). Inhibitors of bacterial immune systems: discovery, mechanisms and applications. Nature Reviews Genetics, doi: 10.1038/s41576-023-00676-9.
McDonnell, B., Mahony, J., Hanemaaijer, L., Kouwen, T. R. H. M., & van Sinderen, D. (2018). Generation of bacteriophage-insensitive mutants of Streptococcus thermophilus via an antisense RNA CRISPR-Cas silencing approach. Applied and Environmental Microbiology, 84(4), doi: 10.1128/AEM.01733-17.
Mendoza, S. D., Nieweglowska, E. S., Govindarajan, S., Leon, L. M., Berry, J. D., Tiwari, A., Chaikeeratisak, V., Pogliano, J., Agard, D. A., & Bondy-Denomy, J. (2020). A bacteriophage nucleus-like compartment shields DNA from CRISPR nucleases. Nature, 577(7789), 244-248, doi: 10.1038/s41586-019-1786-y.
Mesyanzhinov, V. V., Robben, J., Grymonprez, B., Kostyuchenko, V. A., Bourkaltseva, M. V., Sykilinda, N. N., Krylov, V. N., & Volckaert, G. (2002). The genome of bacteriophage φKZ of Pseudomonas aeruginosa. Journal of Molecular Biology, 317(1), 1-19, doi: 10.1006/jmbi.2001.5396.
Moelling, K., Broecker, F., Russo, G., & Sunagawa, S. (2017). RNase H as gene modifier, driver of evolution and antiviral defense. Frontiers in Microbiology, 8, 1745, doi: 10.3389/fmicb.2017.01745.
Morgan, C. J., Enustun, E., Armbruster, E. G., Birkholz, E. A., Prichard, A., Forman, T., Aindow, A., Wannasrichan, W., Peters, S., Inlow, K., Shepherd, I. L., Razavilar, A., Chaikeeratisak, V., Adler, B. A., Cress, B. F., Doudna, J. A., Pogliano, K., Villa, E., Corbett, K. D., & Pogliano, J. (2024). An essential and highly selective protein import pathway encoded by nucleus-forming phage. Proceedings of the National Academy of Sciences of the United States of America, 121(19), e2321190121, doi: 10.1073/pnas.2321190121.
Mozumdar, D., Fossati, A., Stevenson, E., Guan, J., Nieweglowska, E., Rao, S., Agard, D., Swaney, D. L., & Bondy-Denomy, J. (2024). Characterization of a lipid-based jumbo phage compartment as a hub for early phage infection. Cell Host & Microbe, 32(7), 1050-1058.e7. doi: 10.1016/j.chom.2024.05.016.
Mueser, T. C., Hinerman, J. M., Devos, J. M., Boyer, R. A., & Williams, K. J. (2010). Structural analysis of bacteriophage T4 DNA replication: a review in the Virology Journal series on bacteriophage T4 and its relatives. Virology Journal, 7, 359, doi: 10.1186/1743-422X-7-359.
Naknaen, A., Samernate, T., Saeju, P., Nonejuie, P., & Chaikeeratisak, V. (2024). Nucleus-forming jumbophage PhiKZ therapeutically outcompetes non-nucleus-forming jumbophage Callisto. iScience, 27(5), 109790, doi: 10.1016/j.isci.2024.109790.
Neri, U., Wolf, Y. I., Roux, S., Camargo, A. P., Lee, B., Kazlauskas, D., Chen, I. M., Ivanova, N., Zeigler Allen, L., Paez-Espino, D., Bryant, D. A., Bhaya, D., RNA Virus Discovery Consortium, Krupovic, M., Dolja, V. V., Kyrpides, N. C., Koonin, E. V., & Gophna, U. (2022). Expansion of the global RNA virome reveals diverse clades of bacteriophages. Cell, 185(21), 4023-4037.e18, doi: 10.1016/j.cell.2022.08.023
Nielsen, P. E. (2010). Gene targeting and expression modulation by peptide nucleic acids (PNA). Current Pharmaceutical Design, 16(28), 3118-3123, doi: 10.2174/138161210793292546.
Nieweglowska, E. S., Brilot, A. F., Méndez-Moran, M., Kokontis, C., Baek, M., Li, J., Cheng, Y., Baker, D., Bondy-Denomy, J., & Agard, D. A. (2023). The ϕPA3 phage nucleus is enclosed by a self-assembling 2D crystalline lattice. Nature Communications, 14(1), 927, doi: 10.1038/s41467-023-36526-9.
Ofir, G., & Sorek, R. (2018). Contemporary Phage Biology: From Classic Models to New Insights. Cell, 172(6), 1260-1270, doi: 10.1016/j.cell.2017.10.045.
Pifer, R., & Greenberg, D. E. (2020). Antisense antibacterial compounds. Translational Research: The Journal of Laboratory and Clinical Medicine, 223, 89-106, doi: 10.1016/j.trsl.2020.06.001.
Piya, D., Nolan, N., Moore, M.L., Ramirez Hernandez, L.A., Cress, B.F., Young, R., Arkin, A.P., and Mutalik, V.K. (2023). Genome-wide CRISPRi knockdown to map gene essentiality landscape in Coliphages λ and P1. bioRxiv, 2023.05.14.540688. 10.1101/2023.05.14.540688.
Popella, L., Jung, J., Do, P. T., Hayward, R. J., Barquist, L., & Vogel, J. (2022). Comprehensive analysis of PNA-based antisense antibiotics targeting various essential genes in uropathogenic Escherichia coli. Nucleic Acids Research, 50(11), 6435-6452, doi: 10.1093/nar/gkac362.
Putzeys, L., Wicke, L., Brand5o, A., Boon, M., Pires, D. P., Azeredo, J., Vogel, J., Lavigne, R., & Gerovac, M. (2024). Exploring the transcriptional landscape of phage-host interactions using novel high-throughput approaches. Current Opinion in Microbiology, 77, 102419, doi: 10.1016/j.mib.2023.102419.
Prichard, A., Lee, J., Laughlin, T.G., Lee, A., Thomas, K.P., Sy, A.E., Spencer, T., Asavavimol, A., Cafferata, A., Cameron, M., et al. (2023). Identifying the core genome of the nucleus-forming bacteriophage family and characterization of Erwinia phage RAY. Cell Reports 42, 112432.
Prichard, A., & Pogliano, J. (2024). The intricate organizational strategy of nucleus-forming phages. Current Opinion in Microbiology, 79, 102457, doi: 10.1016/j.mib.2024.102457.
Robins, W. P., Faruque, S. M., & Mekalanos, J. J. (2013). Coupling mutagenesis and parallel deep sequencing to probe essential residues in a genome or gene. Proceedings of the National Academy of Sciences of the United States of America, 110(9), E848-E857, doi: 10.1073/pnas.1222538110.
Ren, P., Chen, T., Liu, N., Sun, W., Hu, G., Yu, Y., Yu,B., Ouyang, P., Liu, D., & Chen, Y. (2020) Efficient Biofilm-Based Fermentation Strategies by eDNA Formation for I-Proline Production with Corynebacterium glutamicum. ACS Omega 5 (51), 33314-33322; DOI: 10.1021/acsomega.0c05095
Robinson, J. T., Thorvaldsdóttir, H., Winckler, W., Guttman, M., Lander, E. S., Getz, G., & Mesirov, J. P. (2011). Integrative genomics viewer. Nature Biotechnology, 29(1), 24-26, doi: 10.1038/nbt.1754.
Skinner, S. O., Sepúlveda, L. A., Xu, H., & Golding, I. (2013). Measuring mRNA copy number in individual Escherichia coli cells using single-molecule fluorescent in situ hybridization. Nature Protocols, 8(6), 1100-1113, doi: 10.1038/nprot.2013.066.
Sprenger, M., Siemers, M., Krautwurst, S., & Papenfort, K. (2024). Small RNAs direct attack and defense mechanisms in a quorum sensing phage and its host. Cell Host & Microbe, 32(5), 727-738.e6, doi: 10.1016/j.chom.2024.03.010.
Stead, M. B., Agrawal, A., Bowden, K. E., Nasir, R., Mohanty, B. K., Meagher, R. B., & Kushner, S. R. (2012). RNAsnap™: a rapid, quantitative and inexpensive, method for isolating total RNA from bacteria. Nucleic Acids Research, 40(20), e156-e156, doi: 10.1093/nar/gks680.
Sturino, J. M., & Klaenhammer, T. R. (2002). Expression of antisense RNA targeted against Streptococcus thermophilus bacteriophages. Applied and Environmental Microbiology, 68(2), 588-596, doi: 10.1128/AEM.68.2.588-596.2002.
Vogel, J. (2020). An RNA biology perspective on species-specific programmable RNA antibiotics. Molecular Microbiology, 113(3), 550-559, doi: 10.1111/mmi. 14476.
Vogele, K., Falgenhauer, E., von Schönberg, S., Simmel, F.C., and Pirzer, T. (2021). Small antisense DNA-based gene silencing enables cell-free bacteriophage manipulation and genome replication. ACS Synthetic Biology 10(3), 459-465, doi: 10.1021/acssynbio.0c00402.

## Claims

1. A method for assaying for the relevance of a protein of a phage for the replication of said phage in a bacterium, comprising the steps of (a) contacting said bacterium either before, concomitant with or after infection with said phage, in particular before infection with said phage, with at least one compound CPP-ASO^{p}, wherein CPP is a cell-penetrating peptide and ASO^{p} is an antisense construct that is specifically binding to an mRNA coding for said protein, and (b) evaluating at least one parameter that is a measure for said phage replication.

2. The method according to claim 1, wherein said bacterium is a Gram-negative bacterium, particularly a Gammaproteobacterium, particularly a Gammaproteobacterium selected from the Enterobacteriaceae, Vibrionaceae, or Pseudomonadaceae family, particularly selected from *Escherichia coli* and *Pseudomonas aeruginosa* species.

3. The method according to claim 2, wherein said CPP is selected from the list of (KFF)3K, (RXR)4XB, (RFF)3R, (RXR)4, (RFR)4XB, (RFF)3R(3'), (RX)6B, pip1(D), drosocin, oncocin, TAT, BF2A, BF2A-RXR, drosocin-RXR, (KFF)3K(D), pip1, DAB, DAP, drosocin(D), (P59→W59)-Tat48-60, ANT, P12-(CH2)6, B12-SS, Bac1-15, IsCT-p, K6L2W3, KLW-L9,13-a, NLS-Gb3, Pep-1-K, SA-3, TDN, and TPk, in particular is selected from (KFF)3K and (RXR)4XB, more particularly is (RXR)4XB.

4. The method according to claim 2 or 3, wherein said phage is selected from: *Chimalliviridae,* in particular a Chimallivirus selected from ΦKZ, KTN4, EL, 201ϕ2-1, and ΦPA3, or a non-nucleus-forming jumbo phage, in particular PA5Oct, when the bacterium is *Pseudomonas aeruginosa,* or a Goslar Chimallivirus, when the bacterium is *Escherichia coli;* an enterobacteria phage λ, when the bacterium is *Escherichia coli;* and a single-stranded RNA phage, in particular a PP7 phage, when the bacterium is *Pseudomonas aeruginosa,* or a levivirus, when the bacterium is *Escherichia coli,* in particular a Levivirus selected from MS2 and Qβ.

5. The method according to claim 1, wherein said bacterium is a Gram-positive bacterium, particularly an actinobacterium or a firmicute, particularly selected from *Streptococcus, Staphylococcus* and *Lactobacillus,* particularly *Lactobacillus casei, Lactococcus bulgaricus* and *Streptococcus thermophilus.*

6. The method according to claim 5, wherein said CPP is selected from the list of (KFF)3K, TAT (RXR)4XB, (RFR)4XB, ANT, K8, pip1 and pip1(D), in particular is selected from (KFF)3K and (RXR)4XB, more particularly is (KFF)3K.

7. The method according to claim 5 or 6, wherein said phage is selected from phage PL-1 and phage phi FSW, more particularly is phage PL-1, when said bacterium is *Lactobacillus casei;* or wherein said phage is selected from cos, pac, 5093, and 987 group *Siphoviridae* family phages, when said bacterium is *Streptococcus thermophilus.*

8. The method according to any one of claims 1 to 7, wherein said ASO^{p} is selected from DNA, RNA, peptide nucleic acids (PNAs), peptide-conjugated phosphorodiamidate morpholino oligomers (PPMOs), phosphorothioate (PTO)-modified DNA, 2'-methylated RNA (RNA-OMe), 2'-methoxyethylated RNA (RNA-MOE), 2'-fluorinated RNA (RNA-F), 2'-4'-bridged RNA (BNA), and 2'-4'-locked RNA (LNA), in particular is selected PNAs, PPMOs, PTO-modified DNA, RNA-Ome, RNA-MOE, RNA-F, BNA, and LNA, more particularly from PNAs and PPMOs, most particularly is a PNA.

9. The method according to claim 8, wherein said ASO^{p} is a PNA consisting of n nucleotides, wherein n is selected from 9, 10, 11, 12, 13, 14, and 15, in particular wherein n is selected from 9, 10, 11, 12, and 13, more particularly wherein n is selected from 10, 11, and 12, in particular wherein n is 10 or 11.

10. The method of claim 8 or 9, wherein said ASO^{p} is specific for a region of the gene comprising the start codon and/or the ribosomal binding site.

11. The method according to any one of claims 1 to 10, wherein said at least one parameter is selected from plaque-forming units, colony-forming units, formation of intracellular structures, early, intermediate and late phage transcripts, transcriptome and proteome signatures.

12. The method according to claim 11, wherein said phage is a Chimallivirus and wherein said at least one parameter is the formation of an intracellular structure selected from an EPI vesicle and a phage nucleus.

13. The method according to any one of claims 1 to 12, wherein said step (a) is performed using one or more of the following conditions: culturing of said bacterium in MH medium; culturing said bacterium to a cell density OD600 of 0.3; performing a pre-incubation step with said CPP-ASO^{p} of up to 30 min, in particular, wherein the pre-incubation time does not end more than 5 minutes before infection with said phage; using a concentration of said CPP-ASO^{p} of at least 2 µM, in particular between 2 and 10 µM, more particularly between 4 and 6 µM; infecting said bacterium with said phage at a multiplicity of infection (MOI) of 0.0001; using a time of spotting after infection of 210 min; using about 3 replication rounds and nearly full lysis of cells; and using a length of 10 to 11 nucleotides for the ASO^{p}.

14. The method according to any one of claim 1 to 13, wherein said protein is a protein having a putative role selected from a polymerase, a ribonuclease, capsid, endolysin, terminase, import proteins, and phylogenetically conserved proteins.

15. A method for assaying for the relevance of a protein of a bacterium for the replication of a phage in said bacterium, comprising the steps of (a) contacting said bacterium either before, concomitant with or after infection with said phage with at least one compound CPP-ASO^{b}, wherein CPP is a cell-penetrating peptide and ASO^{b} is an antisense construct that is specifically binding to an mRNA coding for said protein, and (b) evaluation at least one parameter that is a measure for said replication.

16. A method for inhibiting the formation of progeny particles of a phage from a bacterium infected with said phage, comprising the step of contacting said bacterium with at least one compound CPP-ASO^{p}, wherein CPP is a cell-penetrating peptide and ASO^{p} is an antisense construct that is specifically binding to an mRNA coding for a protein of said phage that is essential or important for phage replication, and/or at least one compound CPP-ASO^{b}, wherein ASO^{b} is an antisense construct that is specifically binding to an mRNA coding for a protein of said bacterium that is essential or important for phage replication.

17. A method for preventing the formation of phage progeny particles from a bacterium at risk of being infected with said phage, comprising the step of contacting said bacterium with at least one compound CPP-ASO^{p}, wherein CPP is a cell-penetrating peptide and ASO^{p} is an antisense construct that is specifically binding to an mRNA coding for a protein of said phage that is essential or important for phage replication, and/or at least one compound CPP- ASO^{b}, wherein ASO^{b} is an antisense construct that is specifically binding to an mRNA coding for a protein of said bacterium that is essential or important for phage replication.

18. The method according to claim 17, wherein said bacterium is *Pseudomonas aeruginosa,* wherein said phage is a Chimallivirus ΦKZ and wherein said protein of said phage is selected from ΦKZ049, picA (ΦKZ069), ΦKZ042, ΦKZ155, ΦKZ056, ΦKZ174,ΦKZ177 and ΦKZ186, in particular wherein said bacterium is used in a biofilm-based fermentation.

19. A method for promoting the formation of phage progeny particles from a bacterium comprising the steps of (a) contacting said bacterium with at least one compound CPP- ASO^{b}, wherein CPP is a cell-penetrating peptide and ASO^{b} is an antisense construct that is specifically binding to an mRNA coding for a protein essential or important for said bacterium's defence system directed against replication of said phage in said bacterium; and (b) infecting said bacterium with said phage.

20. The method according to claim 19, wherein said bacterium is *Pseudomonas aeruginosa,* wherein said phage is a Chimallivirus ΦKZ and wherein said protein is jukA and/or jukB.

21. A method for promoting the formation of phage progeny particles from a bacterium infected with, or otherwise comprising, a phage, in particular a prophage, comprising the steps of (a) contacting said bacterium with at least one compound CPP-ASO^{p/b}, wherein CPP is a cell-penetrating peptide and ASO^{p/b} is an antisense construct that is specifically binding to an mRNA coding for a phage or bacterial protein, respectively, repressing, preventing or delaying replication of said phage in said bacterium.

22. The method of claim 21, wherein said step (a) results in lysis of said bacterium.

23. A compound CPP-ASO^{p}, wherein CPP is a cell-penetrating peptide and ASO^{p} is an antisense construct that is specifically binding to an mRNA coding for a protein repressing, preventing or delaying replication of a phage, in particular a lytic phage, in a bacterium for use in the treatment of a patient suffering from an infection with said bacterium.
